(19) Europäisches Patentamt
European Patent Office
Office européen des brevets

(11) EP 1 645 565 B1

# (12) EUROPEAN PATENT SPECIFICATION

(45) Date of publication and mention of the grant of the patent:
**18.02.2009 Bulletin 2009/08**

(51) Int Cl.:
***C07K 7/64*** *(2006.01)* ***A61K 38/13*** *(2006.01)*

(21) Application number: **05024216.3**

(22) Date of filing: **08.10.1998**

## (54) Deuterated cyclosporine analogs and their use as immunomodulating agents

Deuterierte Cyclosporin-analoga und ihre Verwendung als immunmodulierende Agenzien

Analogue de la cyclosporine deuterié et luers utilisations comme agents immunomodulés

(84) Designated Contracting States:
**AT BE CH CY DE DK ES FI FR GB GR IE IT LI LU MC NL PT SE**
Designated Extension States:
**RO**

(30) Priority: **08.10.1997 US 61360 P**

(43) Date of publication of application:
**12.04.2006 Bulletin 2006/15**

(62) Document number(s) of the earlier application(s) in accordance with Art. 76 EPC:
**98947718.7 / 0 991 660**

(73) Proprietor: **Isotechnika Inc.**
**Edmonton,**
**AB T6E 6W2 (CA)**

(72) Inventors:
- **Naicker, S.Selveraj**
  **Edmonton, Alberta T6J3J4 (CA)**
- **Foster, Robert T.**
  **Edmonton, Alberta T6J1X9 (CA)**
- **Yatscoff, Randall W.**
  **Edmonton, Alberta T6R 2Z5 (CA)**

(74) Representative: **Reverzani, Cristina et al**
**Haseltine Lake**
**Theatinerstrasse 3**
**80333 München (DE)**

(56) References cited:
**WO-A-95/26325**

- **D. SEEBACH ET AL.: "MODIFICATION OF CYCLOSPORIN A (CS): GENERATION OF AN ENOLATE AT THESARCOSINE RESIDUE AND REACTIONS WITH ELECTROPHILES" HELVETICA CHIMICA ACTA, vol. 76, 1993, pages 1564-1590, XP002022424**
- **KOBEL H ET AL: "Contribution to knowledge of the biosynthesis of cyclosporin A" EXPERIENTIA, BIRKHAUSER VERLAG. BASEL, CH, vol. 39, 1983, pages 873-876, XP002090783 ISSN: 0014-4754**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 645 565 B1

## Description

### REFERENCE TO RELATED APPLICATION

This application is a continuation-in-part of U.S. provisional application serial no. 60/061,360 filed October 8, 1997 which is relied upon and incorporated in its entirety.

### INTRODUCTION AND BACKGROUND

Cyclosporin derivatives of the present invention are disclosed which possess enhanced efficacy and reduced toxicity over naturally occurring and other presently known cyclosporins and cyclosporine derivatives. The cyclosporin derivatives of the present invention are produced by chemical and isotopic substitution of the cyclosporine A (CsA) molecule by:

1. Chemical substitution and optionally deuterium substitution of amino acid 1; and
2. Deuterium substitution at key sites of metabolism of the cyclosporine A molecule such as amino acids 1, 4, 9.

The most active derivatives of the invention were those possessing both chemical and deuterium substitution.

The cyclosporins are a family of, neutral, hydrophobic cyclic undecapeptides, containing a novel nine-carbon amino acid (MeBmt) at position 1 of the ring that exhibit potent immunosuppressive, antiparasitic, fungicidal, and chronic anti-inflammatory properties. The naturally occurring members of this family of structurally related compounds are produced by various fungi imperfecti. Cyclosporines A and C, are the major components. Cyclosporine A, which is discussed further below, is a particularly important member of the cyclosporin family of compounds. Twenty four minor metabolites, also oligopeptides, have been identified: Lawen et al, J. Antibiotics 42, 1283 (1989); Traber et al, Helv. Chim. Acta 70, 13 (1987); Von Wartburg and Traber Prog. Med. Chem., 25, 1 (1988).

Isolation of cyclosporines A and C, as well as the structure of A were reported by A. Ruegger et al., Helv. Chim. Acta 59, 1075(1976); M. Dreyfuss et al., J. Appl. Microbiol. 3, 125 (1976). Crystal and molecular structures of the iodo derivative of A have been reported by T. J. Petcher et al., Helv. Chim. Acta 59, 1480 (1976). The structure of C was reported by R. Traber et al., ibid. 60, 1247 (1977). Production of A and C has been reported by E. Harri et al., U.S. Pat. No. 4,117,118 (1978 to Sandoz). Isolation, characterization and antifungal activity of B, D, E, as well as the structures of A through D have been reported by R. Traber et al., Helv. Chim. Acta 60, 1568(1977). Isolation and structures of E, F, G, H, I: eidem, ibid. 65, 1655 (1982). Preparation of [2-Deutero-3-fluoro-D-Ala]$^8$-CsA is disclosed by Patchett et al in GB 2,206,199A which was published on Dec. 29, 1988.

Cyclosporin was discovered to be immunosuppressive when it was observed to suppress antibody production in mice during the screening of fungal extracts. Specifically, its suppressive effects appear to be related to the inhibition of T-cell receptor-mediated activation events. It accomplishes this by interrupting calcium dependent signal transduction during T-cell activation by inactivating calmodulin and cyclophilin, a peptidly propyl isomerase. It also inhibits lymphokine production by T-helper cells in vitro and arrests the development of mature CD8 and CD4 cells in the thymus. Other in vitro properties include inhibition of IL-2 producing T-lymphocytes and cytotoxic T-lymphocytes, inhibition of IL-2 released by activated T-cells, inhibition of resting T-lymphocytes in response to alloantigen and exogenous lymphokine, inhibition of IL-1 production, and inhibition of mitogen activation of IL-2 producing T-lymphocytes, Further evidence indicates that the above effects involve the T-lymphocytes at the activation and maturation stages.

Stimulation of TCR (T cell receptor) by foreign antigen on a major histocompatibility (MHC) molecule on the surface of the T cell results in the activation of a TCR signal transmission pathway (exact method of transmission unknown) through the cytoplasm causing the signal results in the activation of nuclear transcription factors, i.e. nuclear factors of activated T-cells (NF-AT) which regulate transcription of T-cell activation genes. These genes include that of lymphokine interleukin-2 (IL-2). Translation of the message is followed by secretion of IL-2. T-cell activation also involves the appearance of the lymphokine receptor IL-2R on the cell srface. After IL-2 binds to IL-2R, a lymphokine receptor (LKR) signal transmission pathway is activated. The immunosuppressive drug, rapamycin, inhibits this pathway.

CsA is a potent inhibitor of TCR-mediated signal transduction pathway. It inhibits binding of NF-AT to the IL-2 enhancer, and thus inhibits transcriptional activation. CsA binds to cyclophilin, which binds to calcineurin, which is a key enzyme in the T-cell signal transduction cascade.

Cyclophilin is found in prokaryotic and eukarotic organisms and is ubiquitous and abundant. Cyclophilin is a single polypeptide chain with 165 amino acid residues. It has a molecular mass of 17.8 kD. A roughly spherical molecule with a radius of 17 angstroms, cyclophilin has a eight-stranded antiparallel beta barrel. Inside the barrel, the tightly packed core contains mostly hydrophobic side chains. CsA has numerous hydrophobic side chains which allow it to fit into the cyclophilin beta barrel. Cyclophillin catalyzes the interconversion of the cis and trans-rotamers of the peGIFdyl-prolyl amide bond of peptide and protein substrates. Cyclophilin is identical in structure with peptidyl prolyl cis-trans

isomerase and bears structural resemblance to the superfamily of proteins that transports ligands such as retinol-binding protein (RBP). These proteins carry the ligand in the barrel core. But cyclophilin actually carries the ligand binding site on the outside of the barrel. The tetrapeptide ligand binds in a long deep groove on the protein surface between one face of the beta barrel and the Thr1 16-Gly130 loop.

Further properties have also been reported in studies of the biological activity of CsA: J. F. Borel et al., Agents Actions 6, 468 (1976). Pharmacology: Eidem. Immunology 32, 1017 (1977); R. Y. Calne, Clin. Exp. Immunol. 35, 1 (1979). Human studies: R. Y. Calne et al., Lancet 2, 1323(1978); R. L. Powles et al., ibid. 1327; R. L. Powles et al., ibid 1, 327 (1980). In vitro activity (porcine T-cells): D. J. White et al., Transplantation 27, 55 (1979). Effects on human lymphoid and myeloid cells: M. Y. Gordon, J. W. Singer, Nature 279, 433(1979). Clinical study of CsA in graft-versus-host disease: P. J. Tutschka et al., Blood 61, 318(1983).

## Mechanism of Cyclosporine A Action

### Cyclosporine A-Cyclophilin A complex

CsA, as discussed above, binds to the cyclophilin beta barrel. Thirteen CyP A residues define the CsA binding site. These residues are Arg 55, Phe 60, Met 61, Gln 63, Gly 72, Ala 101, Asn 102, Ala 103, Gln 111, Phe 113, Trp 121, Leu 122, His 126. The largest side-chain movements are 1.3 A for Arg 55 and up to 0.7 A for Phe 60, Gin 63, and Trp 121. There are four direct hydrogen bonds between the CyP A and CsA. Residues 4, 5, 6, 7, 8 of CsA protrude out into the solvent and are thought to be involved in binding the effector protein, calcineurin (Pflugl, G., Kallen, J., Schirmer, T., Jansonius, J.N., Zurini, M.G.M., & Walkinshaw, M.D. (1993) Nature 361, 91-94.)

### Function of CsA-CyP A complex.

The CsA-CyP A complex inhibits the phosphatase activity of the heterodimeric protein serine/threonine phosphatase or calcineurin (Liu, J., Farmer, J.D., Lane, W.S., Friedman, J., Weissman, I., & Schreiber, S.L. (1991) Cell 66, 807-15.; Swanson, S.K., Born, T., Zydowsky, C.D., Cho, H., Chang, H.Y., & Walsh, C.T. (1992) Proc. Natl. Acad. Sci.USA 89, 3686-90). CyP A binds CsA with an affinity of ca. 10 nM. The complex is then presented to calcineurin (Liu, J., Farmer, J.D., Lane, W.S., Friedman, J., Weissman, I., & Schreiber, S.L. (1991) Cell 66, 807-15.).

Calcineurin dephosphorylates the transcription factor NFAT found in the cytoplasm of T-cells. Dephosphorylation allows NFAT to translocate to the nucleus, combine with jun/ fos genes and activate the transcription of the IL-2 gene responsible for cell cycle progression, leading to immune response. CsA-CyP A complex inhibits the phosphatase activity of calcineurin and ultimately immunosuppression (Etzkorn, F. A., Chang, Z., Stolz, L.A., &Walsh, C.T. (1994) Biochemistry 33, 2380-2388.). Neither CsA or CyP A alone are important immunologically. Only their complex is important (Liu, J., Farmer, J.D., Lane, W.S., Friedman, J., Weissman, I., & Schreiber, S.L. (1991) Cell 66, 807-15).

### Metabolism of Cyclosporine:

Cyclosporine is metabolized in liver, small intestine and kidney to more than 30 metabolites. The structure of 13 metabolites and 2 phase 11 metabolites have been identified and at least 23 further metabolites have been isolated by HPLC and their structures characterized by mass spectrometry. The reactions involved in phase I metabolism of cyclosporine are hydroxylation, demethylation as well as oxidation and cyclisation at amino acid 1. Several clinical studies and reports showed an association between blood concentrations of cyclosporine metabolites and neuro- or nephrotoxicity. In vitro experiments indicate that metabolites are considerably less immunosupressive and more toxic than CsA.

As exemplified by the ever expanding list of indications for which CsA has been found useful, the cyclosporin family of compounds find utility in the prevention of rejection or organ and bone marrow transplants; and in the treatment of psoriasis, and a number of autoimmune disorders such as type 1 diabetes mellitus, multiple sclerosis, autoimmune uveitis, and rheumatoid arthritis. Additional indications are discussed infra.

As is generally accepted by those of skill in the art, inhibition of secretion of interleukin-2 (IL-2) and other lymphokines from lymphocytes, is a useful indicator of intrinsic immunosuppressive activity of a cyclosporin analog. For a recent review of cyclosporin uses and mechanisms of action see Wenger et al Cyclosporine: Chemistry, Structure-Activity Relationships and Mode of Action, Progress in Clinical Biochemistry and Medicine, vol. 2, 176 (1986).

Cyclosporin A is a cyclic peptide which contains several N-methyl amino acids and, at position-8, contains a D-alanine. The structure of Cyclosporin A[a] is given below:

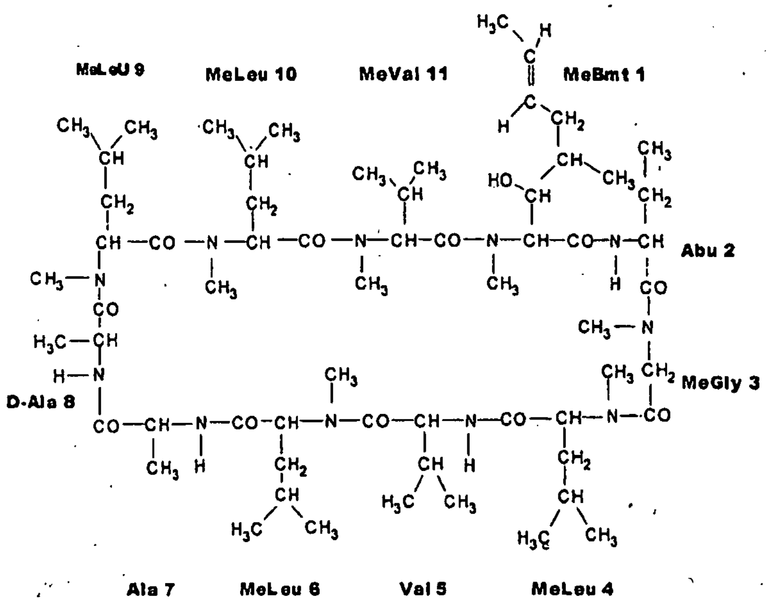

[a]Unless otherwise specified, each of the amino acids of the disclosed cyclosporin is of the L-configuration.

As is the practice in the field, a particular cyclosporin analog may be named using a shorthand notation identifying how the analog differs from cyclosporin A. Thus, cyclosporin C which differs from cyclosporin A by the threonine at position-2 may be identified as $[Thr]^2$-cyclosporin or $[Thr]^2$-CsA. Similarly, cyclosporin B is $[Ala]^2$-CsA; cyclosporin D is $[Val]^2$-CsA; cyclosporin E is $[Val]^{11}$-CsA; cyclosporin F is $[3\text{-DesoxyMeBmt}]^1$-CsA; cyclosporin G is $[NVa]^2$-CsA; and cyclosporin H is $[D\text{-MeVal}]^{11}$-CsA.

D-Serine and D-Threonine have been introduced into the 8-position of cyclosporin A by biosynthesis resulting in active compounds. See R. Traber et al. J. Antibiotics 42, 591 (1989). D-Chloroalanine has also been introduced into position-8 of Cyclosporin A by biosynthesis. See A. Lawen et al *J. Antibiotics* 52, 1283 (1989).

**Indications for Cyclosporine Therapy**

Immunoregulatory abnormalities have been shown to exist in a wide variety of autoimmune and chronic inflammatory diseases, including systemic lupus erythematosis, chronic rheumatoid arthritis, type 1 diabetes mellitus, inflammatory bowel disease, biliary cirrhosis, uveitis, multiple sclerosis and other disorders such as Crohn's disease, ulcerative colitis, bullous pemphigoid, sarcoidosis, psoriasis, ichthyosis, and Graves ophthalmopathy. Although the underlying pathogenesis of each of these conditions may be quite different, they have in common the appearance of a variety of autoantibodies and self-reactive lymphocytes. Such self-reactivity may be due, in part, to a loss of the homeostatic controls under which the normal immune system operates.

Similarly, following a bone marrow or an organ transplantation, the host lymphocytes recognize the foreign tissue antigens and begin to produce antibodies which lead to graft rejection.

One end result of an autoimmune or a rejection process is tissue destruction caused by inflammatory cells and the mediators they release. Anti-inflammatory agents, such as NSAID's (Non-Steroidal Anti-inflammatory Drugs), and corticosteroids act principally by blocking the effect of, or secretion of, these mediators, but do nothing to modify the immunologic basis of the disease. On the other hand, cytotoxic agents, such as cyclophosphamide, act in such a nonspecific fashion that both the normal and autoimmune responses are shut off. Indeed, patients treated with such nonspecific immunosuppressive agents are as likely to succumb to infection as they are to their autoimmune disease.

Generally, a cyclosporin, such as cyclosporine A, is not cytotoxic nor myelotoxic. It does not inhibit migration of monocytes nor does it inhibit granulocytes and macrophage action. Its action is specific and leaves most established immune responses intact. However, it is nephrotoxic and is known to cause the following undesirable side effects:

(1) abnormal liver function;
(2) hirsutism;
(3) gum hypertrophy;
(4) tremor;
(5) neurotoxicity;

(6) hyperaesthesia; and
(7) gastrointestinal discomfort.

A number of cyclosporines and analogs have been described in the patent literature:

U.S. Pat. No. 4,108,985 issued to Ruegger, et al. on Aug. 22, 1978 entitled, "Dihydrocyclosporin C", discloses dihydrocyclosporin C, which can be produced by hydrogenation of cyclosporin C.

U.S. Pat. No. 4,117,118 issued to Harri, et al. on Sep. 26, 1978 entitled, "Organic Compounds", discloses cyclosporins A and B, and the production thereof by fermentation.

U.S. Pat. No. 4,210,581 issued to Ruegger, et al. on Jul. 1, 1980 entitled, "Organic Compounds", discloses cyclosporin C and dihydrocyclosporin C which can be produced by hydrogenation of cyclosporin C.

U.S. Pat. No. 4,220,641, issued to Traber, et al. on Sep. 2, 1980 entitled, "Organic Compounds", discloses cyclosporin D, dihydrocyclosporin D, and isocyclosporin D.

U.S. Pat. No. 4,288,431 issued to Traber, et al. on Sep. 8, 1981 entitled, "Cyclosporin Derivatives, Their Production and Pharmaceutical Compositions Containing Them", discloses cyclosporin G, dihydrocylosporin G, and isocyclosporin G.

U.S. Pat. No. 4,289,851, issued to Traber, et al. on Sep. 15, 1981 entitled, "Process for Producing Cyclosporin Derivatives", discloses cyclosporin D, dihydrocyclosporin D, and isocyclosporin D, and a process for producing same.

U.S. Pat. No. 4,384,996, issued to Bollinger, et al. on May 24, 1983 entitled "Novel Cyclosporins", discloses cyclosporins having a β-vinylene-α-amino acid residue at the 2-position and/or a β-hydroxy-α-amino acid residue at the 8-position. The cyclosporins disclosed included either MeBmt or dihydro-MeBmt at the I-position

U.S. Pet. No. 4,396,542, issued to Wenger on Aug. 2, 1983 entitled, "Method for the Total Synthesis of Cyclosporins, Novel Cyclosporins and Novel Intermediaires and Methods for their Production", discloses the synthesis of cyclosporins, wherein the residue at the I-position is either MeBmt, dihydro-MeBmt, and protected intermediaires.

U.S. Pet. No. 4,639,434, issued to Wenger, et al on Jan 27, 1987, entitled "Novel Cyclosporins", discloses cyclosporins with substituted residues at positions J, 2, 5 and 8.

U.S. Pat. No. 4,681,754, issued to Siegel on Jul. 21, 1997 entitled, "Counteracting Cyclosporin Organ Toxinity", discloses methods of use of cyclosporin comprising co-dergocrine.

U.S. Pat. No. 4,703,033 issued to Seebach on Oct 27, 1987 entitled, "Novel Cyclosporins", discloses cyclosporins with substituted residues at positions 1, 2 and 3. The substitutions at position-3 include halogen.

H. Kobel and R. Traber, Directed Biosynthesis of Cyclosporins, European J. Appln. Microbiol Biotechnol., 14, 237B240 (1982), discloses the biosynthesis of cyclosporins A, B, C, D & G by fermentation.

Additional cyclosporin analogs are disclosed in U.S. Pat No. 4,798,823, issued to Witzel, entitled. New Cyclosporin Analogs with Modifed "C-9 amino acids", which disclosed cyclosporine analogs with sulfur-containing amino acids at position-1.

D. Seebach at al., Helvetica Chimica Acta, vol. 76, p. 1564-1590 (1993) describe a process for introducing side chains into peptides, and in particular in position 3 of cyclosporin A. Kobel H. et al., Experientia. Birkhauser Verlag, Basel, vol. 39, p. 873-876 (1983) describe $^{3}$H- and $^{13}$C-NMR spectroscopic investigations on the structure of labelled cyclosporin A. WO-95/26325 discloses methods of enhancing the efficacy of drugs like nifedipine by deuteration.

## SUMMARY OF THE INVENTION

The present invention concerns chemically substituted and deuterated analogs of cyclosporine A and related cyclosporines.

An object of the present invention is to provide new cyclosporine analogs which have enhanced efficacy end altered pharmacokinetic and pharmacodynamic parameters. Another object of the present invention is to provide a cyclosporine analog for the care of immunoregulatory disorders and diseases, including the prevention, control and treatment thereof. An additional object of the present invention is to provide pharmaceutical compositions for administering to a patient in need of the treatment one or more of the active immunosuppressive agents of the present invention. Still a further object of this Invention is to provide a method of controlling graft rejection, autoimmune and chronic inflammatory diseases by administering a sufficient amount of one or more of the novel immununosuppressive agents in a mammalian species in need of such treatment. Finally, it is the object of this invention to provide processes for the preparation of the active compounds of the present invention.

Substitution and deuteration of the cyclosporine molecule results in altered physicochemical and pharmacokinetic properties which enhance its usefulness in the treatment of transplantation rejection, host vs. graft disease, graft vs. host disease, a plastic anemia, focal and segmental glomerulosclerosis, myasthenia gravis, psoristic arthritis, relapsing polychondritis and ulcerative colitis.

Embodiments of the invention include CsA derivatives wherein one or more hydrogen atoms in the I, 3 and 9

amino acid positions are substituted with a deuterium atom and wherein the cyclosporine A derivatives are optionally chemically substituted at the amino acid 9 position. A further specific embodiment of the invention is the CsA derivative represented by formula I:

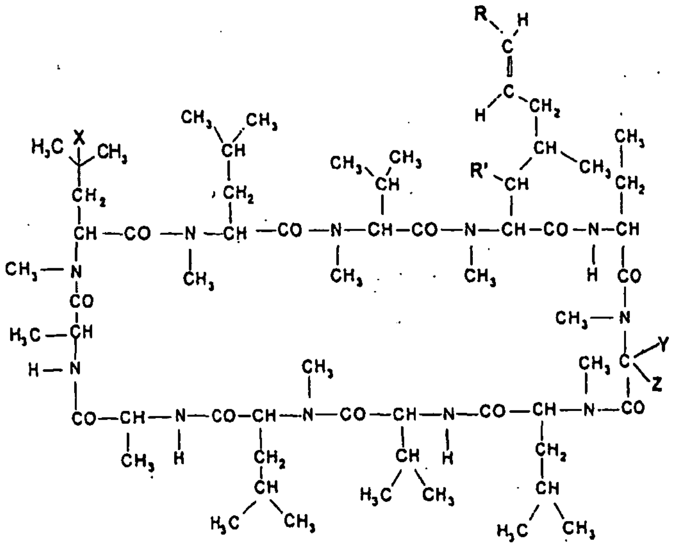

(I)

where R is (i) a deuterium or (ii) a saturated or unsaturated straight or branched aliphatic chain of from 2 to 16 carbon atoms and containing one or more deuteriem atoms or an ester, ketone or alcohol of the carbon chain and optionally containing one or more substituents selected from halogen, nitro, amino, amido, aromatic, and heterocyclic, or (iii) R is an aromatic or heterocyclic group optionally containing a deuterium atom, and X, Y, and Z are hydrogen or deuterium provided that at least one of X, Y or Z is deuterium and R' is an OH or an ester or is an O and together with a carbon adjacent to a double bond on amino acid I form a heterocyclic ring such as 5-membered rings where the heteroatom is oxygen. Other specific embodiments of the present invention include the CsA derivative of formula I where R is a saturated or unsaturated carbon chain of from 2 to 3 carbons containing one or more deuterium. Further specific embodiments include those of formulas 5g and 5e below:

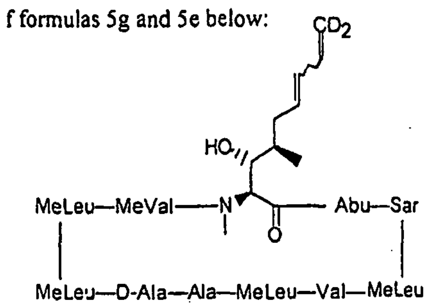


(5g)

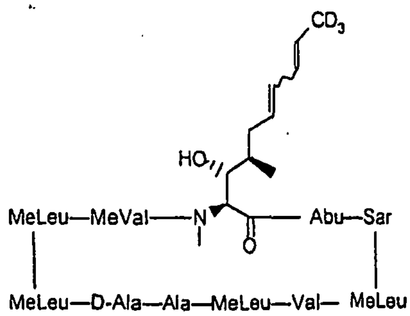

(5e)

## DESCRIPTION OF THE FIGURE

Figure 1 is the structure of cyclosporine A showing a site of deuteration at the amino acid 3 position.
Figure 2 is the structure of cyclosporine A showing a site of deuteration at the amino acid 9 position.
Figure 3 is scheme I of the synthesis of the cyclosporine derivatives.
Figure 4 is scheme II of the synthesis of the cyclosporine derivatives.
Figure 5 is a graph of the results of the calcineurin assay of Example 9.
Figure 6 is a graph of the results of a mixed lymphocyte reaction assay of Example 10.

## DETAILED DESCRIPTION OF THE INVENTION

Substitution of deuterium for ordinary hydrogen and deuterated substrates for protio metabolites can produce profound changes in biosystems. Isotopically altered drugs have shown widely divergent pharmacological effects. Pettersen et al., found increased anti-cancer effect with deuterated 5,6-benzylidene-dl-L-ascorbic acid (Zilascorb) [Anticancer Res. 12, 33 (1992)].

Substitution of deuterium in methyl groups of cyclosporine will result in a slower rate of oxidation of the C-D bond relative to the rate of oxidation of a non-deuterium substituted C-H bond. The isotopic effect acts to reduce formation of demethylated metabolites and thereby alters the pharmacokinetic parameters of the drug. Lower rates of oxidation, metabolism and clearance result in greater and more sustained biological activity. Deuteration is targeted at various sites of the cyclosporin molecule to increase the potency of drug, reduce toxicity of the drug, reduce the clearance of the pharmacologically active moiety and improve the stability of the molecule.

## Isotopic Substitution:

Stable isotopes (e.g., deuterium, $^{13}C$, $^{15}N$, $^{18}O$) are nonradioactive isotopes which contain one additional neutron than the normally abundant isotope of the respective atom. Deuterated compounds have been used in pharmaceutical research to investigate the in vivo metabolic fate of the compounds by evaluation of the mechanism of action and metabolic pathway of the non deuterated parent compound. (Blake et al. J. Pharm. Sci. 64, 3, 367-391,1975). Such metabolic studies are important in the design of safe, effective therapeutic drugs, either because the *in vivo* active compound administered to the patient or because the metabolites produced from the parent compound prove to be toxic or carcinogenic (Foster et al., Advances in Drug Research Vol. 14, pp. 2-36, Academic press, London, 1985).

Incorporation of a heavy atom particularly substitution of deuterium for hydrogen, can give rise to an isotope effect that could alter the pharmacokinetics of the drug. This effect is usually insignificant if the label is placed at a metabolically inert position of the molecule.

Stable isotope labeling of a drug can alter its physico-chemical properties such as pKa and lipid solubility. These changes may influence the fate of the drug at different steps along its passage through the body. Absorption, distribution, metabolism or excretion can be changed. Absorption and distribution are processes that depend primarily on the molecular size and the lipophilicity of the substance. These effects and alterations can affect the pharmacodynamic response of the drug molecule if the isotopic substitution affects a region involved in a ligand-receptor interaction.

Drug metabolism can give rise to large isotopic effect if the breaking of a chemical bond to a deuterium atom is the rate limiting step in the process. While some of the physical properties of a stable isotope-labeled molecule are different from those of the unlabeled one, the chemical and biological properties are the same, with one important exception: because of the increased mass of the heavy isotope, any bond involving the heavy isotope and another atom will be stronger than the same bond between the light isotope and that atom. In any reaction in which the breaking of this bond is the rate limiting step, the reaction will proceed slower for the molecule with the heavy isotope due to "kinetic isotope effect" A reaction involving breaking a C-D bond can be up to 700 per cent slower than a similar reaction involving breaking a C-H bond. If the C-D bond is not involved in any of the steps leading to the metabolite , there may not be any effect to alter the behavior of the drug. If a deuterium is placed at a site involved in the metabolism of a drug , an isotope effect will be observed only if breaking of the C-D bond is the rate limiting step There is evidence to suggest that whenever cleavage of an aliphatic C-H bond occurs, usually by oxidation catalyzed by a mixed-function oxidase, replacement of the hydrogen by deuterium will lead to observable isotope effect. It is also important to understand that the incorporation of deuterium at the site of metabolism slows its rate to the point where another metabolite produced by attack at a carbon atom not substituted by deuterium becomes the major pathway a process called "metabolic switching". It is also observed that one of the most important metabolic pathways of compounds containing aromatic systems is hydroxylation leading to a phenolic group in the 3 or 4 position to carbon substituents. Although this pathway involves cleavage of the C-H bond, it is often not accompanied by an isotope effect, because the cleavage of this bond mostly not involved in the rate

limiting step. The substitution of hydrogen by deuterium at the stereo center will induce a greater effect on the activity of the drug.

## Synthesis of Cyclosporine Derivatives:

The staring material for the preparation of the compounds of this invention is cyclosporine A. The process for preparing the compounds of the present invention are illustrated.as shown in *scheme 1* in figure 3. It will be readily apparent to one of ordinary skill in the art reviewing the synthetic route depicted below that other compounds with formula 1 can be synthesized by substitution of appropriate reactants and agents in the synthesis shown below.

The first step in the process for making deuterated cyclosporin analogs is the preparation of the key intermediate 3 and 6 . This can be achieved by the oxidation of the double bond in the amino acid 1. Treatment of cyclosporin with acetic anhydride and excess of dimethylaminopyridine provided the hydroxyl protected acetyl cyclosporin. 2 .Although cleavage of the double bond could then be accomplished by treatment of **2** with ozone, or $KMnO_4$/ $NaIO_4$, it was found out that $O_SO_4/NaIO_4$ was the reagent of choice for the transformation to the aldehyde product **3**. The reaction was generally found to be cleaner, producing the required material and to proceed in higher yield. The drawback to this reaction is that $OsO_4$, is expensive and highly toxic, so that its use is limited. But the results can be accomplished more economically by the use of $H_2O$, with $OsO_4$ present in catalytic amounts, t-butyl hydroxide in alkaline solution and N-methylmorpholine-N-oxide can be substituted for $H_2O_2$ in this process. The aldehyde compound 3 was further treated with various deuterated alkyl or aryl triphenyl phosphonium derivatives(wittig reagents) and hydrolysis by alkaline solution provided the final derivatives ( **5 a-h**), We also developed a general procedure to obtain various compounds as shown in *Scheme II* in figure 4.

In this approach , the aldehyde derivative 3 was treated with the Wittig reagent prepared by using standard procedure. The resultant product on mild acid hydrolysis provided the key intermediate aldehyde product **6** This was further treated with second deuterated alkyl or aryl triphenylphosphonium halide reagents and on mild acid hydrolysis yielded the required products. This method provides control over the extension of the diene system. By using this approch, olefinic double bonds can be introduced step by step.

A third approach to prepare the deuterated compounds 5a-h-- is by heating non deuterated cyclosporin analogs described earlier, in a deuerated solvent such as deuterated water, deuterated acetic acid in the presence of acid or base catalyst.

Preferred cyclosporins of the present invention are those which both contain a deuterium and a chemical substitution on amino acid 1 such as those of formula 11:

(II) 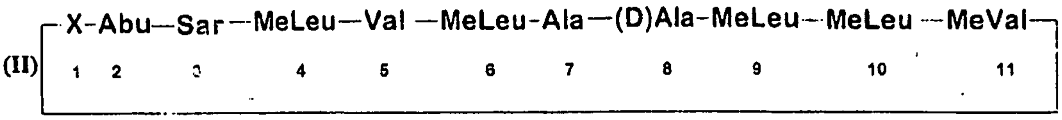

Where X is

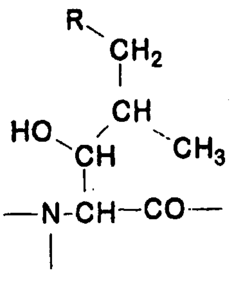

And R= --CHO, -CDO, $-CH=CD-CD_3$, $-CD=CD-CD_3$,$-CH=CH-CH=CD-CD_3$ $-CD=CH-CD=CD.CD_3$, $-CH=CH-CH=CD_2$, $-CD=CH-CD=CD_2$,$-CH=CD_2$,$-CH=CH_2$ and $-CD=CD_2$.

**EXAMPLES**:

**Example 1**.

To a stirred solution of cyclosporine **1**(1.01g, 0.84mmol) in acetic anhydride (20mL) at room temperature was added DMAP (150mg, 1.23mmol, 1.5eq). After stirring overnight, the reaction mixture was partitioned between EtOAc (50ml) and water (25ml). The separated EtOAc layer was then washed with water (50mL) and brine (50mL), dried ($MgSO_4$) and the solvent removed *in vacuo* to give the crude product as a glassy solid. Purification by flash chromatography through a short column of silica (2% MeOH/DCM) and lyophilisation from benzene yielded 2 (1.044g, 0.84mmol, quant.) as a fluffy, colourless solid; $[\alpha]_D^{25}$ -305.7 (c. 0.3, $CHCl_3$); $\nu_{max}$ ($CHCl_3$ cast)/$cm^{-1}$ 3328m, 2963m, 1746m, 1627s, 1528m, 1472m, 1233m; $\delta_H$ (600MHz, $C_6D_6$) 8.73 (1H, d, *J* = 9.5Hz, NH), 8.30 (1H, d, *J* = 7.0Hz, NH), 7.92 (1H, d, *J* = 7.5Hz, NH), 7.49 (1H, d, *J* = 7.5Hz, NH), 6.05 (1H, d, *J* = 11.5Hz), 5.88 (1H, dd, *J* = 3.5, 11.5Hz), 5.82 (1H, d, *J* = 11.5Hz), 5.65 (1H, dd, *J* = 4.0, 12.0Hz), 5.60 (1H, dd, *J* = 3.5, 12.5Hz), 5.63-5.57 (1H, m), 5.51-5.45 (1H, m), 5.37 (1H, dd, *J* = 5.5, 8.5Hz), 5.05-5.01 (2H, complex), 4.99 (1H, d, *J* = 11.0Hz), 4.76 (1H, p, *J* = 7.0Hz), 4.58 (1H, p, *J* = 7.0Hz), 4.02 (1H, d, *J* = 13.5Hz), 3.47 (3H, s), 3.30 (3H, s), 3.17 (3H, s), 3.11 (3H, s), 2.98 (3H, s), 2.68-2.62 (1H, m), 2.63 (3H, s), 2.51-2.39 (2H, complex), 2.34-2.25 (8H, complex), 2.03 (3H, s), 1.97-1.85 (2H, complex), 1.83 (3H, dd, *J* = 1.0, 6.5Hz), 1.82-1.77 (2H, complex), 1.68-1.61 (3H, complex), 1.55 (3H, d, *J* = 7.0Hz), 1.55-1.51 (1H, m), 1.44-1.38 (1H, m), 1.32-1.20 (5H, complex), 1.29 (3H, d, *J* = 7.0Hz), 1.21 (3H, d, *J* = 6.5Hz), 1.17 (3H, d, *J* = 6.5Hz), 1.14 (3H, d, *J* = 6.5Hz), 1.08 (3H, d, *J* = 6.5Hz), 1.04 (3H, d, *J* = 6.0Hz), 1.03 (3H, d, *J* = 7.0Hz), 1.00 (3H, d, *J*= 7,OHz), 0.93 (3H, d, *J* = 6.0Hz), 0.92 (3H, d, *J* = 6.5Hz), 0.88-0.84 (9H, complex), 0.76 (3H, d, *J*= 6,5Hz), 0.57 (3H, d, *J*= 6.5Hz); $\delta_c$ (75MHz, $C_6D_6$) 173.6, 173.2, 172.8, 172.6, 171.3, 171.1, 170.71, 170.67, 170.4, 170.2, 169.8, 167.9 (C=0), 129.0, 126.2 (C=C), 73.1 (COAc), 58.1, 57.1, 56.0, 55.0, 54.6, 54.2, 50.3, 49.9, 48.6, 48.1, 47.8, 44.5, 40.8, 39.1, 35.7, 33.6, 32.9, 32.1, 31.5, 31.2, 30.0, 29.7, 29.5, 29.3, 24.9, 24.6, 24.4, 24.0, 23.6, 23.4, 23.3, 21.7, 21.1, 21.0, 20.6, 20.3, 19.5, 18.5, 18.0, 17.7, 17.5, 17.4, 14.9, 9.7; *m*/*z* (Electrospray)

**Example 2**

To a solution of compound **2** (289mg, 0.23mmol) in a 1:1 mixture of dioxane and water (5mL) was added firstly sodium metaperiodate (100mg, 0.47mmol, 2eq) and secondly a solution of osmium tetraoxide (5mL; 0.5g $O_sO_4$ in 250mL of solvent). Two-phase work-up, purification by flash column chromatography (40% acetone in petroleum ether) and lyophilisation from benzene gave compound **3**. (226mg, 0.18mmol, 80%) as a fluffy, colourless solid; $[\alpha]_D^{25}$ -260.0 (c. 0.1, $CHCl_3$); $\nu_{max}$ ($CHCl_3$ cast)/$cm^{-1}$ 3325m, 2962m, 1748w, 1724w, 1677m, 1626s, 1228m, 755m; $\delta_H$ (300MHz, $C_6D_6$) 8.63 (1H, d, *J* = 9.5Hz, NH), 8.16 (1H, d, *J*= 7.0Hz, NH), 7.95 (1H, d, *J* = 7.5Hz, NH), 7.48 (1H, d, *J* = 9.0Hz, NH), 5.93 (1H, d, *J* = 7.5Hz), 5.84 (1H, dd, *J* = 4.0, 11.5Hz), 5.70 (1H, d, *J* = 11.5Hz), 5.56-5.54 (1H, m), 5.32 (1H, dd, *J* = 5.5, 8.0Hz), 5.07-4.88 (3H, complex), 4.72 (1H, p, *J* = 7.0Hz), 4.49 (1H, p, *J* = 7.0Hz), 3.98 (1H, d, *J* = 14.0Hz), 3.42 (3H, s, $CH_3N$), 3.27 (3H, s, $CH_3N$), 3.12 (3H, s, $CH_3N$), 3.07 (3H, s, $CH_3N$), 2.91 (3H, s, $CH_3N$), 2.79 (3H, s, $CH_3N$), 2.59 (3H, s, $CH_3N$), 2.42-2.08 (10H, complex), 1.94 (3H, s, $CH_3CO_2$), 1.47 (3H, d, *J*= 7.0Hz), 1.24 (3H, 7.0Hz), 1.14-1.09 (9H, complex), 1.04 (3H, d, *J* = 6.5Hz), 1.01 (3H, d, *J* = 7.0Hz), 0.96 (3H, d, *J* = 6.5Hz), 0.92 (3H, d, *J* = 6.5Hz), 0.91 (3H, d, *J* = 6.5Hz), 0.89 (3H, d, *J* = 6.0Hz), 0.83 (6H, d, *J* = 6.5Hz), 0.74 (3H, d, *J* = 6.5Hz), 0.59 (3H, d, *J* = 6.5Hz); $\delta_c$ (75MHz, $C_6D_6$) 202.5 (CHO), 174.4, 174.0, 173.7, 172.8, 171.6, 171.5, 171.2, 171.1, 170.6, 170.2, 170.2, 168.1, 73.0, 58.7, 57.6, 56.7, 55.5, 55.0, 54.5, 49.4, 48.9, 48.5, 48.1, 45.0, 44.6, 41.3, 39.8, 38.8, 37.7, 36.2, 32.5, 32.0, 31.6, 30.9, 30.3, 30.0, 29.8, 29.6, 25.6, 25.3, 25.0, 24.8, 24.5, 24.0, 23.8, 23.4, 22.0, 21.7, 21.2, 20.5, 20.0, 19.8, 18.8, 18.5, 18.2, 17.4, 15.2, 10.0;M/z(Electrospray) 1232.8 ($MH^+$, 100%).

**Example 3**

Method A: To a solution of compound **3**( 315mg, 0.26mmol) in THF (5mL) at 0°C was added a solution of the deuterophosphorus ylid (2.67mmol, ~10eq), prepared from $d_5$-ethyltriphenylphosphonium iodide. After work-up, purification by flash column chromatography (30% to 60% acetone in PE) and HPLC (60% to 65% MeCN in water), then lyophilisation from benzene yielded compound **4** (153mg, 0.12mmol, 47%) as a fluffy, colourless solid.

Method B: To a stirred solution of compound 3 (287mg, 0.23mmol) in THF (5mL) under Ar at -78°C was carefully added a solution of phosphorus ylid (formed by the addition of sodium hexamethyldisilylamide (1.0M; 2.25mL,

2.25mmol, ~10eq) to a suspension of $d_5$-ethyltriphenylphosphonium iodide (480mg, 1.13mmol, ~5eq) in THF (10 mL) under Ar at room temperature). After stirring for 2hr with gradual warming to room temperature, the reaction mixture was cooled to 0°C and was quenched by the addition of 10% AcOH/THF (10 mL). The reaction mixture was concentrated *in vacuo* and partitioned between water (20mL) and EtOAc (20mL). The aqueous layer was further extracted with EtOAc (20mL) and the combined organic extracts were then washed with IN HCl (20mL) and water (20mL), dried ($MgSO_4$) and the solvent removed *in vacuo* to give the crude product. Purification by flash column chromatography (40% acetone in petroleum ether) and lyophilisation from benzene yielded compound **4d** (84mg, 67μmol, 29%) as a fluffy, colourless solid; $[\alpha]_D^{25}$ -283.0 (c. 0.1, $CHCl_3$); $\nu_{max}$ ($CHCl_3$ cast)/$cm^{-1}$ 3320m, 3010m, 2959s, 2924s, 2871m, 2853m, 1743m, 1626s, 756s; $\delta_H$ (600MHz, $C_6D_6$) 8.78 (1H, d, *J* = 9.5Hz), 8.33 (1H, d, *J* = 7.0Hz), 7.99 (1H, d, *J* = 7.5Hz), 7.59 (1H, d, *J* = 9.0Hz), 6.09 (1H, d, *J* = 11.5Hz), 5.92 (1H, dd, *J* = 4.0, 11.0Hz), 5.86 (1H, d, *J* = 11.5Hz), 5.72-5.64 (2H, complex), 5.62 (1H, dd, *J* = 3.5, 12.5Hz), 5.40 (1H, dd, *J* = 5.5, 8.5Hz), 5.10-5.02 (3H, complex), 4.80 (1H, q, *J* = 7,0Hz), 4.60 (1H, q, *J* = 7.0Hz), 4.05 (1H, d, *J* = 14.0Hz), 3.51 (3H, s), 3.31 (3H, s), 3.20 (3H, s), 3.13 (3H, s), 3.01 (3H, s), 2.87 (3H, s), 2.64 (3H, s), 2.45 (1H, dt, *J* = 4.0, 12.5Hz), 2.36-2.20 (10H, complex), 2.06 (3H, s), 1.93-1.79 (3H, complex); $\delta_D$ (84MHz, $C_6H_6$)

$\delta_c$ (125MHz, $C_6D_6$) 174.5, 173.7, 173.6, 173.1, 171.7, 171.4, 170.9, 170.7, 170.6, 170.3, 170.0, 168.4, 130.2 (C=C), 123.8 (C=C), 73.8 (MeBmt C-3), 58.7, 58.1, 57.6, 57.1, 55.5, 55.0, 54.5, 49.4, 49.0, 48.6, 48.2, 45.0, 41.4, 39.9, 39.0, 37.8, 34.2, 33.9, 32.6, 32.3, 32.0, 31.4, 30.9, 30.8, 30.2, 30.1, 30.0, 29.9, 29.8, 29.6, 28.5, 25.6, 25.3, 25.0, 24.9, 24.8, 24.1, 23.9, 23.8, 23.6, 23.1, 22.1, 21.7, 21.4, 20.7, 20.0, 19.9, 19.8, 18.9, 18.7, 18.6, 18.3, 17.4, 15.3, 14.3, 10.2; *m*/*z* (Electrospray) 1270 ([M+Na]$^+$, 100%), 1286 ([M+K]$^+$, 20).

**Example 4**

To a stirred solution of **4d** (84mg, 67μmol) in MeOH (5mL) and water (2.5mL) at room temperature was added potassium carbonate (99mg, 0.72mmol, ~10eq). After stirring overnight, the MeOH was removed *in vacuo* and the aqueous residue was partitioned between EtOAc (10mL) and 5% citric acid solution (10mL). The EtOAc layer was then washed with water (10mL) and brine (10mL), dried ($MgSO_4$) and the solvent removed *in vacuo* to give the crude product. HPLC purification (60% to 65% MeCN in water) and lyophilisation from benzene yielded compound **5d** (59mg, 49μmol, 70%) as a fluffy, colourless solid; $[\alpha]_D^{25}$ - 262.0 (c. 0.05, $CHCl_3$); $\nu_{max}$ ($CHCl_3$ cast)/$cm^{-1}$ 3318m, 3008m, 2960s, 2872m, 1627s, 1519m, 1470m, 1411m, 1295m, 1095m, 754m; $\delta_H$ (600MHz, $C_6H_6$) 8.27 (1H, d, *J* = 9.5Hz), 7.96 (1H, d, *J* = 7.5Hz), 7.63 (1H, d,J= 8.0Hz), 7.45 (1H, d, *J* = 9.0Hz), 5.87 (1H, dd, *J* = 3.5, 11.0Hz), 5.74 (1H, d, *J* = 7.5Hz), 5.73-5.69 (1H, m), 5.66-5.64 (1H, br d, *J* = 11.0Hz), 5.79 (1H, dd, *J* = 4.0, 11.5Hz), 3.39 (1H, dd, *J* = 5.5, 10.5Hz), 5.33 (1H, dd, *J* = 5.5, 8.5Hz), 5.24 (1H, d, *J* = = 11.0Hz), 5.12 (1H, dt, *J* = 7.5, 10.0Hz), 4.88-4.79 (3H, complex), 4.22 (1H, dd, *J* = 5.5, 7.5Hz), 4.00 (1H, d, 13.5Hz), 3.72 (3H, s), 3.22 (3H, s), 3.06 (3H, s), 2.97 (3H, s), 2.92 (3H, s), 2.85 (3H, s), 2.67-2.60 (1H, m), 2.58 (3H, s), 2.56-2.50 (1H, br m), 2.33-2.23 (4H, complex), 2.20-2.07 (4H, complex), 1.80-1.74 (3H, complex), 1.67 (3H, d, *J* = 7.0Hz), 1.56-1.50 (2H, complex), 1.46-1.23 (9H, complex), 1.17-1.13 (16H, complex), 1.06 (3H, d, *J* = 6.5Hz), 1.02 (3H, d, *J* = 7.0Hz), 0.98 (3H, d, *J* = 6.5Hz), 0.96 (3H, d, *J* = 7.0Hz), 0.92-0.89 (9H complex), 0.86 (3H, t, *J* = 7.5Hz), 0.83 (3H, d, *J* = 6.0Hz), 0.64 (3H, d, *J* = 6.5Hz); $\delta_D$ (84MHz, $C_6H_6$) 1.64 ($C\underline{D}_3$); $\delta_c$ (75MHz, $C_6H_6$) 174.2, 174.1, 174.0, 173.7, 171.8, 171.4, 171.2, 170.5, 170.4, 170.3, 169.8, 130.2, 124.1, (99.2,) 74.3, (67.1,) 66.3, 66.1, 61.0, 59.5, 58.3, 57.8, 55.7, 55.5, 55.4, 49.4, 49.0, 48.4, 45.3, 41.4, 39.6, 39.0, 37.8, 36.5, 36.1, 35.8, 33.7, 31.6, 30.8, 30.4, 30.1, 29.9, 29.5, 29.4, 25.5, 25.2, 25.0, 24.9, 24.5, 24.2, 23.8, 23.7, 23.6, 22.0, 21.4, 20.0, 18.8, 18.5, 17.8, 16.0, 10.1; *m*/*z* (Electrospray) 1206 ([M+H]$^+$, 30%), 1228 ([M+Na]$^+$, 100), 1244 ([M+K]$^+$, 25).

**Example 5**

To a vigorously stirred mixture of compound **3** (49mg, 39.8μmol) and deuterated $d_3$-allyltriphenylphosphonium bromide (311mg, 812μmol, ~20eq) in benzene (3mL) at room temperature was added 1N NaOH (3mL). Stirring was continued at room temperature for 5days, after which time the 2 layers were separated, the benzene layer was washed with water (5mL), dried ($MgSO_4$) and the solvent removed *in vacuo* to give the crude product. Purification by HPLC (20% to 60% MeCN in water) and lyophilisation from benzene yielded compound **4g** (23mg, 18.3μmol, 47%) as a fluffy, colourless solid; $[\alpha]_D^{25}$ -264.2 (c. 0.24, $CHCl_3$); $\nu_{max}$ ($CHCl_3$ cast)/$cm^{-1}$ 3322m, 2959m, 1744m, 1626s, 1231m, 754m; $\delta_H$ (300MHz, $C_6D_6$) complex due to 1:1 ratio of geometrical isomers 8.73 (d, *J* = 9.5Hz, N$\underline{H}$), 8.72 (d, *J* = 9.5Hz, N$\underline{H}$), 8.29 (d, *J* = 6.5Hz, N$\underline{H}$), 8.26 (d, *J* = 6.5Hz, N$\underline{H}$), 7.92 (d, *J* = 7.5$\underline{H}$z, N$\underline{H}$), 7.86 (d, *J* = 7.5Hz, N$\underline{H}$), 7.53 (d, *J* = 9.0Hz,

N<u>H</u>), 7.49 (d, *J* = 9.0Hz, N<u>H</u>), 7.10-6.70 (complex), 6.33 (br t, *J* = 11.0Hz), 6.18 (d, *J* = 10.5Hz), 6.12 (d, *J* = 10.5Hz), 6.05 (d, *J* = 11.0Hz), 6.03 (d, *J* = 11.0Hz), 5.90-5.53 (complex), 5.37 (dd, *J* = 6.0, 8.0Hz), 5.20 (d, *J* = 12.0Hz), 5.14 (d, *J* = 12.0Hz), 5.07-4.97 (complex), 4.80-4.70 (complex), 4.57 (p, *J* = 7.0Hz), 4.02 (d, *J* = 14.0Hz), 4.01 (d, *J* = 14.0Hz), 3.47 (s), 3.46 (s), 3.28 (s), 3.26 (s), 3.16 (s), 3.15 (s), 3.09 (s), 2.97 (s), 2.96 (s), 2.84 (s), 2.62 (s), 2.48-2.23 (complex), 2.05 (s), 2.03 (s), 1.95-1.59 (complex), 1.54 (d, *J* = 7.0Hz), 1.53-0.80 (complex), 0.77 (d, *J* = 6.5Hz), 0.58 (d, *J* = 6.5Hz), 0.57 (d, *J* = 6.5Hz); $\delta_c$ (75MHz, $C_6D_6$) 174.5, 174.0, 173.9, 173.6, 173.5, 173.1, 171.7, 171.6, 171.4, 170.9, 170.8, 170.6, 170.6, 170.3, 169.8, 169.7, 168.4, 137.9, 133.9, 133.5, 132.8, 132.3, 131.6, 130.1, 116.9, 115.0, 73.6, 58.6, 57.6, 57.0, 56.8, 55.7, 55.5, 55.0, 54.9, 54.7, 54.5, 49.4, 48.9, 48.5, 48.2, 48.1, 44.9, 41.5, 39.9, 39.0, 38.9, 37.8, 37.6, 36.6, 36.3, 34.1, 33.7, 32.7, 32.1, 32.0, 31.5, 30.9, 30.7, 30.0, 29.8, 29.6, 25.6, 25.5, 25.3, 25.2, 25.0, 24.9, 24.1, 23.9, 23.7, 23.6, 22.1, 21.7, 21.6, 21.4, 21.3, 20.7, 20.0, 19.9, 18.9, 18.6, 18.3, 17.6, 15.3, 10.2; m/z (Electrospray) 1258.8 ($MH^+$, 100%).

**<u>Example 6</u>**

To a vigorously stirred mixture of compound **<u>3</u>** (56mg, 45.5μmol) and deuterated $d_4$-crotyltriphenylphosphonium bromide (360mg, 907μmol, ~20eq) in benzene (3mL) at room temperature was added IN NaOH (3mL). Stirring was continued at room temperature for 5days, after which time the 2 layers were separated, the benzene layer was washed with water (5mL), dried ($MgSO_4$) and the solvent removed in *vacuo* to give the crude product. Purification by HPLC (20% to 60% MeCN in water) and lyophilisation from benzene yielded compound **<u>4e</u>** (23mg, 18.1μmol, 40%) as a fluffy, colourless solid; $[\alpha]_D^{25}$ -236,0 (c. 0.25, $CHCl_3$); $\nu_{max}$ ($CHCl_3$ cast)/$cm^{-1}$ 3324m, 2959m, 2871m, 1745w, 1626s, 1231m; $\delta_H$ (300MHz, $C_6D_6$) complex due to presence of 4 isomers 8.76 (d, *J*= 6.0Hz), 8.73 (d, *J*= 6.0Hz), 8.29 (d, *J* = 7.0Hz), 7.93 (d, *J* = 7.5Hz), 7.88 (d, *J* = 7.5Hz), 7.53 (d, *J* = 9.5Hz), 7.62-7.31 (1H, complex), 7.16-6.88 (2H, complex), 6.59-6.39 (complex), 6.28 (t, *J*= 11.0Hz), 6.15 (d, *J* = 10.5Hz), 6.09 (d, *J*= 10.5Hz), 6.05 (d, *J*= 11.5Hz), 6.03(d, *J*= 11.5Hz), 5,90-5.82 (complex), 5.68-5.35 (complex), 5.08-4.97 (complex), 4.81-4.72 (complex), 4.63-4.53 (complex), 4.03 (d, *J* = 14.0Hz), 3.47 (s), 3.46 (s), 3.28 (s), 3.26 (s), 3.17 (s), 3.15 (s), 3.09 (s), 2.98 (s), 2.97 (s), 2.83 (s), 2.63 (s), 2.62 (s), 2.71-2.56 (complex), 2.47-2.23 (complex), 2.05 (s), 2.04 (s), 2.03 (s), 2.02 (s), 1.98-0.82 (complex), 0.77 (d, *J* = 6.5Hz), 0.58 (d, *J* = 6.5Hz), 0.58 (d, *J* = 6.5Hz); *m*/*z* (Electrospray) 1273.8 ($MH^+$, 100%).

**<u>Example 7</u>**

To a stirred solution of compound **<u>4</u>**<u>g</u> (20mg, 15.9μmol) in methanol (5mL) and water (1mL) at room temperature was added potassium carbonate (30mg, 217μmol), After stirring overnight, the reaction mixture was partitioned between EtOAc (10mL) and 5% aqueous citric acid (10mL). The aqueous layer was further extracted with EtOAc (5mL), the combined organic layers were then washed with 5% citric acid (10mL) and brine (10mL), dried ($MgSO_4$) and the solvent removed *in vacuo* to give the crude product. Purification by HPLC (65% MeCN) and lyophilisation from benzene yielded compound **5g** (10mg, 8.2μmol, 52%) as a fluffy, colourless solid; $[\alpha]_D^{25}$ -285.2 (c. 0.29, $CHCl_3$); $\nu_{max}$ ($CHCl_3$ cast)/$cm^{-1}$ 3500-3200br, 3319m, 2958m, 2927m, 1626s, 1520m, 1468m, 754m; $\delta_H$ (300MHz, $C_6D_6$) complex due to the presence of 2 isomers 8.25 (d, *J* = 10.0Hz, N<u>H</u>), 8.13 (d, *J* = 10.0Hz, N<u>H</u>), 7.93 (d, *J* = 7.0Hz, N<u>H</u>), 7.84 (d, *J* = 7.0Hz, N*H*), 7.67 (d, *J* = 8.0Hz, N<u>H</u>), 7.61 (d, *J* = 8.0Hz, N<u>H</u>), 7.55 (d, *J* = 8.5Hz, N<u>H</u>), 7.54 (d, *J* = 8.5Hz, N<u>H</u>), 6.84 (t, *J* = 10.5Hz), 6.79 (t, *J* = 10.5Hz), 6.58 (t, *J* = 10.5Hz), 6.52 (t, *J* = 10.5Hz), 6.30-6.14 (complex), 5.88-5.78 (complex), 5.75-5.66 (complex), 5.44-4.74 (complex), 4.22-4.15 (complex), 3.95 (d, *J* = 14.0Hz), 3.93 (d, *J*= 14.0Hz), 3.72 (s), 3.68 (s), 3.19 (s), 3.17 (s), 3.05 (s), 3.03 (s), 2.94 (s), 2.93 (s), 2.89 (s), 2.86 (s), 2.82 (s), 2.81 (s), 2.72-2.53 (complex), 2.55 (s), 2.54 (s), 2.49-2.36 (complex), 2.32-2.03 (complex), 1.81-0.81 (complex), 0.65 (d, *J*= 6.5Hz)), *m*/*z* (Electrospray) 1216.8 ($MH^+$, 100%), 607.9 ($[M+2H]^{2+}$, <u>15</u>).

**<u>Example 8</u>**

To a stirred solution of compound **<u>4e</u>** (18mg, 14.2μmol) in methanol (5mL) and water (1mL) at room temperature was added potassium carbonate (35mg, 254μmol). After stirring overnight, the reaction mixture was partitioned between EtOAc (10mL) and 5% aqueous citric acid (10mL). The aqueous layer was further extracted with EtOAc (5mL), the combined organic layers were then washed with 5% citric acid (10mL) and brine (10mL), dried ($MgSO_4$) and the solvent removed *in vacuo* to give the crude product. Purification by HPLC (65% MeCN) and lyophilisation from benzene yielded compound **<u>5e</u>** (10mg, 8.1μmol, 57%) as a fluffy, colourless solid; $[\alpha]_D^{25}$ -285.5 (c. 0.11, $CHCl_3$); $\delta_H$ (300MHz, $C_6D_6$) complex due to presence of 4 isomers 8.31 (d, *J* = 9.5Hz), 8.28 (d, *J*= 9.5Hz), 8.16 (d, *J* = 9.5Hz), 8.14 (d, *J* = 9.5Hz),

7.96 (d, $J$ = 7.5Hz), 7.95 (d, $J$ = 7.5Hz), 7.86 (d, $J$ = 7.5Hz), 7.85 (d, $J$ = 7.5Hz), 7.63 (d, $J$ = 7.5Hz), 7.59 (d, $J$ = 7.5Hz), 7.50-7.44 (complex), 6.60-6.49 (complex), 6.32-6.11 (complex), 5.88-5.83 (complex), 5.76-5.71 (complex), 5.64-5.22 (complex), 5.17-5.08 (complex), 4.91-4.77 (complex), 4.26-4.18 (complex), 3.99 (d, $J$ = 14.0Hz), 3.97 (d, $J$ = 14.0Hz), 3.74 (s), 3.73 (s), 3.71 (s), 3.69 (s), 3.22 (s), 3.21 (s), 3.20 (s), 3.19 (s), 3.07 (s), 3.06 (s), 3.05 (s), 2.97 (s), 2.96 (s), 2.95 (s), 2.92 (s), 2.91 (s), 2.89 (s), 2.84 (s), 2.83 (s), 2.69-2.07 (complex), 2.58 (s), 2.57 (s), 1.84-0.81 (complex), 0.64 (d, $J$ = 6.5Hz); $m/z$ (Electrospray) 1269.8 ($[M+K]^+$, 5%), 1253.8 ($[M+Na]^+$, 30), 1231.8 ($MH^+$)

## Example 9

The immunosupressive activity was tested for deuterated cyclosporin analogs as described below. Compound 5e and compound 5g are more potent than the parent cyclosporin. Calcineurin activity was assayed using a modification of the method previously described by Fruman et al. (A Proc Natl Acad Sci USA, 1992). Whole blood lysates were evaluated for their ability to dephosphorylate a $^{32}$P-labelled 19 amino acid peptide substrate in the presence of okadaic acid, a phosphatase type 1 and 2 inhibitor. Background phosphatase 2C activity (CsA and okadaic acid resistant activity) was determined and subtracted from each sample, with the assay performed in the presence and absence of excess added CsA. The remaining phosphatase activity was taken as calcineurin activity. The results of the calcineurin assay are presented in figure 5. The results are expressed as means ± the standard error of the mean. The results are plotted as CsA derivative concentration in ug/L versus percentage of calcineurin inhibitation. The structures of the compounds assayed include:

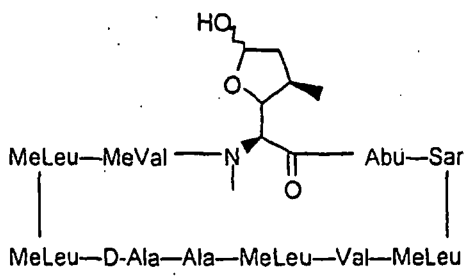

(Compound 2)

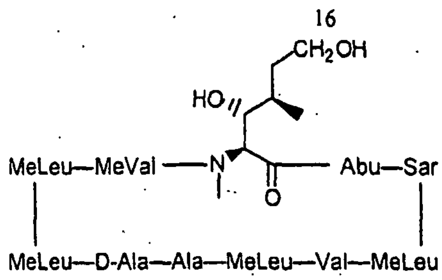

(Compound 3)

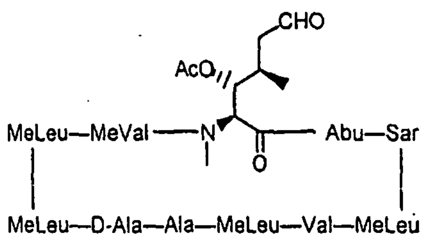

(Compound 6)

**Example 10**

A mixed lymphocyte reaction (MLR) assay was performed with cyclosporine and compounds 5e and 5g. The results are presented in figure 6 and are plotted as the means of four experiments showing concentration of cyclosporine or derivative versus percent inhibition.

The MLR assay is useful for identifying CsA derivatives with biological (immunosuppressive) activity and to quantify this activity relative to the immunosuppressive activity of the parent CsA molecule.

An example of a lymphocyte proliferation assay procedure useful for this purpose is as follows:

1. Collect blood from two individuals (20mls each) and isolate lymphocytes using Ficoll-Paque (Pharmacia Biotech).
2. Count lymphocytes at 1:10 dilution in 2 % acetic acid (v/v).
3. Prepare 10mls of each lymphocyte populations (A + B) at $1\times10^6$ cells/ml in DMEM / 20 % FCS (v/v).
4. Set up a 96 well sterile tissue culture plate, flat bottom (Sarstedt, cat # 83.1835). To each well add:
5. Aliquot 100μl per well lymphocyte population A
6. Aliquot 100μl per well lymphocyte population B
7. Aliquot 20μl per well of drug (CSA and CSA derivatives) at 0, 2.5, 5, 10, 25, 50 and 100μg/L in triplicate in DMEM with no supplements.
8. To measure the effect of drug on proliferation, incubate the plate for 5 days at 37° C in 5 % $CO_2$ atmosphere.
9. On day 6, prepare 3.2mls of 1:50 dilution of Methyl-$^3$H-Thymidine (Amersham Life Science, cat # TRK 120) in DMEM with no supplements. Add 30μl per well and incubate for 18 hours at 37° C in 5 % $CO_2$ atmosphere.
10. On day 7 cells are harvested onto glass microfiber filters GF/A (Whatman, cat # 1820024) using a Cell-Harvestor (Skatron, cat # 11019). Wash cells 3x with 1.0-ml sterile distilled water.
Note: All procedures are done using sterile techniques in a biological flow hood.
11. Place filters in Scintilation vials and add 1.5mls of SciniSafe Plus 50 % scintilation fluid (Fisher, cat # SX-25-5).
12. Measure the amount of radioactivity incorporated in the lymphocytes using a beta counter (Micromedic System Inc., TAURUS Automatic Liquid Scintilation Counter) for 1.0 minute.
13. Calculate averages and standard deviations for each drug and express results as:

$$\% \text{ Inhibition} = \left[1 - \frac{\text{Ave CPM of test drug}}{\text{Ave CPM of zero drug}}\right] \times 100$$

$$\% \text{ Proliferation} = 100 - \% \text{ Inhibition}$$

The MLR assay can be utilized to select antibodies of the invention which bind biologically active CSA metabolites and the parent CSA molecule. Antibodies could also be selected for reactivity to biologically inactive metabolites.

From the results of the calcineurin assay and the mixed lymphocyte reaction assay, it was found that cyclosporines that have been chemically substituted and deuterated at the amino acid 1 position can possess significant immunosupressant activity. In the case of the derivatives 5e and 5g, immunosupressant activity that is significantly greater than CsA was obtained.

**Example 11**

Other cyclosporine derivatives of the invention which have been prepared include the following:

| STRUCTURE | CODE # |
| --- | --- |
| 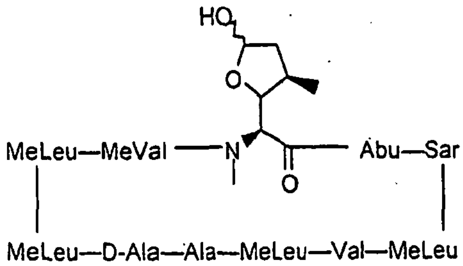 | DB-b1-01 |
| 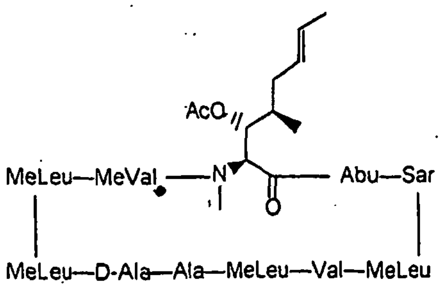 | DB-b1-08 |

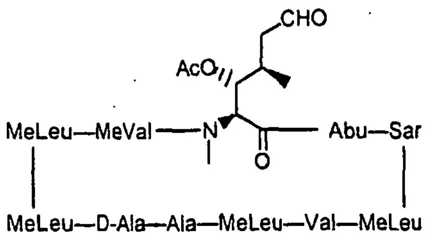

DB1-b1-11

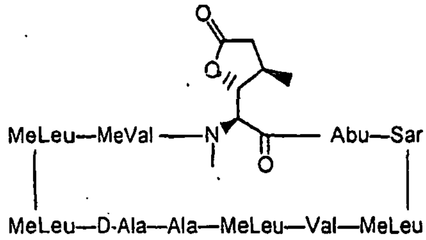

DB1-b1-31

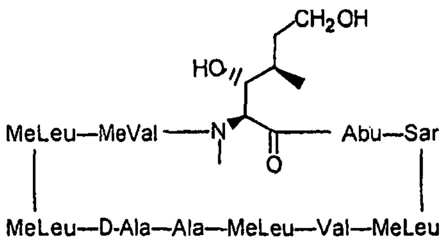

DB1-b1-45

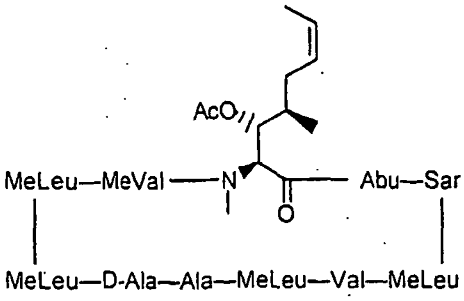

DB-b186C

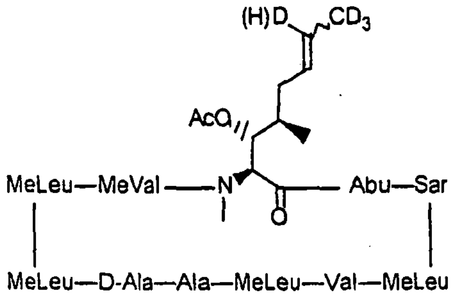

DB-b1-92b

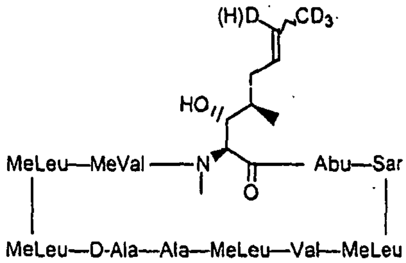

DB-b1-93C

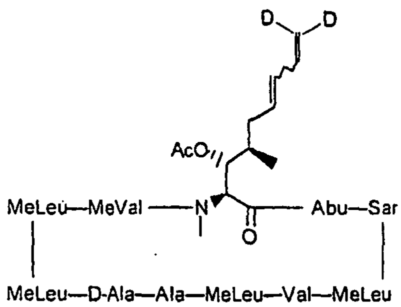

DB-b1-145D

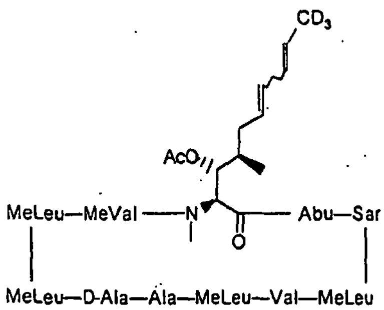

DB-b1-147D

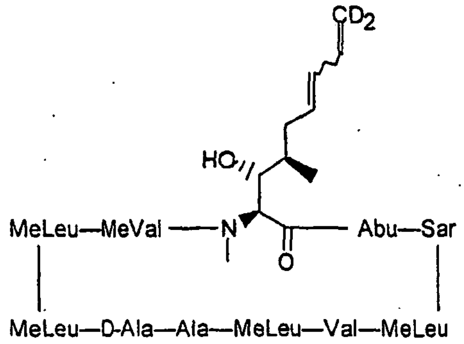

DB-01-148

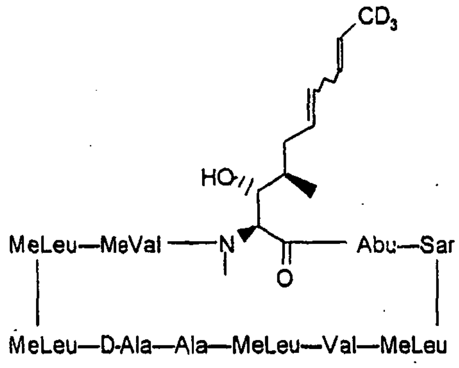

DB-b1-151

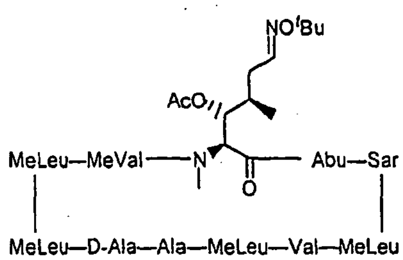

DB-b1-176

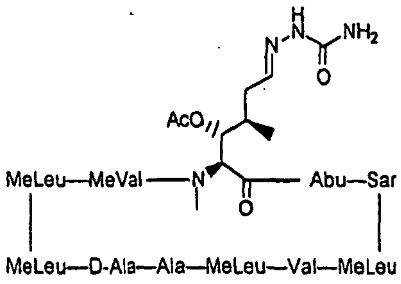

**DB-b1-179**

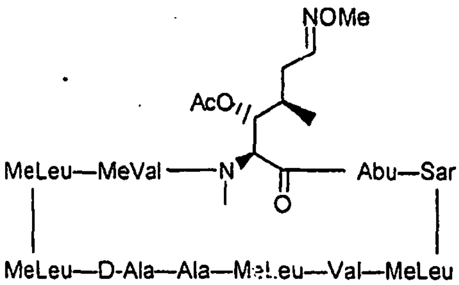

**DB-b1-180**

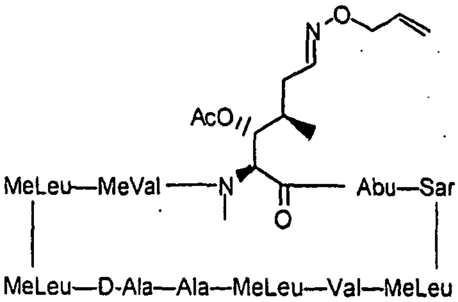

**DB-b1-192**

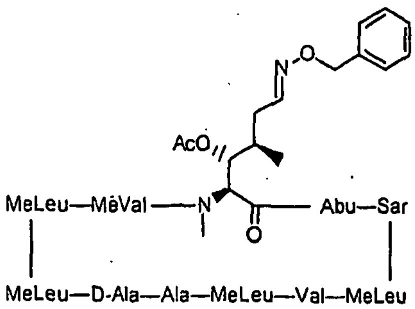

**DB-b1-193**

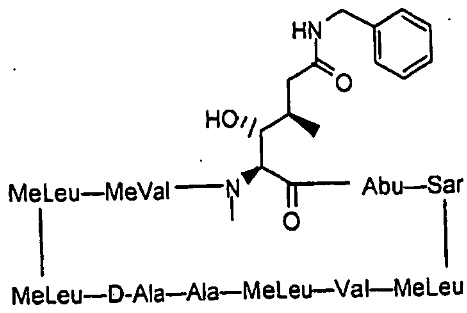

DB-b1-134

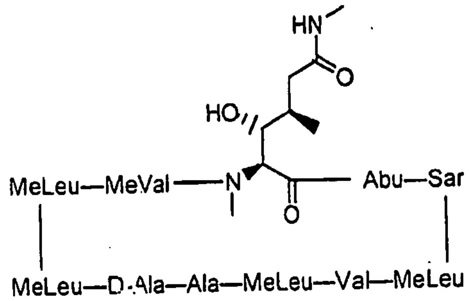

DB-b1-194

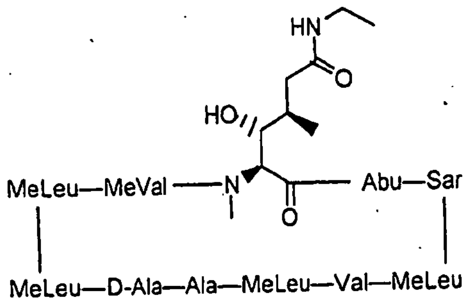

DB-b1-195

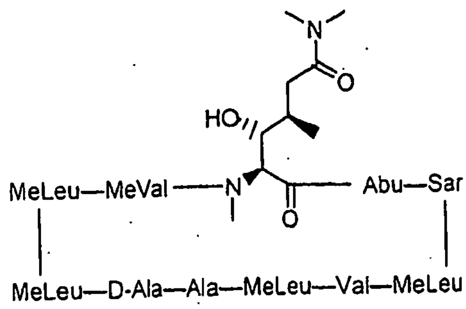

DB-b1-196

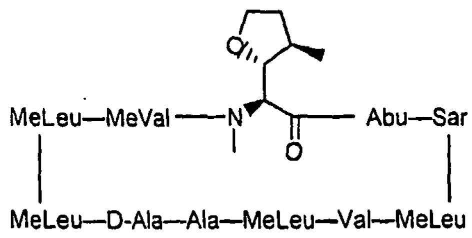

DB-b1-50B

**Drug Composition Formulation and Elicitation of Immunosupression**

Determination of the physicochemical, pharmacodynamic, toxicological and pharmacokinetic properties of the cyclosporine derivatives disclosed can be made using standard chemical and biological assays and through the use of mathematical modeling techniques which are known in the chemical and pharmacological/toxicological arts. The therapeutic utility and dosing regimen can be extrapolated from the results of such techniques and through the use of appropriate pharmacokinetic and/or pharmacodynamic models.

The compounds of this invention may be administered neat or with a pharmaceutical carrier to a warm blooded animal in need thereof. The pharmaceutical carrier may be solid or liquid.

This invention also relates to a method of treatment for patients suffering from immunoregulatory abnormalities involving the administration of the disclosed cyclosporines as the active constituent.

For the treatment of these conditions and diseases caused by immunoirregularity, a deuterated cyclosporin may be administered orally, topically, parenterally, by inhalation spray or rectally in dosage unit formulations containing conventional non-toxic pharmaceutically acceptable carriers, adjuvants and vehicles. The term parenteral, as used herein, includes subcutaneous injections, intravenous, intramuscular, intrasternal injection or infusion techniques.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparation. Tablets containing the active ingredient in admixture with non-toxic pharmaceutically acceptable excipients may also be manufactured by known methods. The excipients used may be for example, (1) inert diluents such as calcium carbonate, lactose, calcium phosphate or sodium phosphate; (2) granulating and disintegrating agents such as corn starch, or alginic acid; (3) binding agents such as starch, gelatin or acacia, and (4) lubricating agents such as magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in the U.S. Pat. Nos. 4,256,108; 4,160,452; and 4,265,874 to form osmotic therapeutic tablets for controlled release.

In some cases, formulations for oral use may be in the form of hard gelatin capsules wherein the active ingredient is mixed with an inert solid diluent, for example, calcium carbonate, calcium phosphate or kaolin. They may also be in the form of soft gelatin capsules wherein the active ingredient is mixed with water or an oil medium, for example peanut oil, liquid paraffin, or olive oil.

Aqueous suspensions normally contain the active materials in admixture with excipients suitable for the manufacture of aqueous suspensions. Such excipients may be

(1) suspending agents such as sodium carboxymethylcellulose, methylcellulose, hydroxypropylmethylcellulose, sodium alginate, polyvinylpyrrolidone, gum tragacanth and gum acacia;
(2) dispersing or wetting agents which may be

   (a) a naturally-occurring phosphatide such as lecithin,
   (b) a condensation product of an alkylene oxide with a fatty acid, for example, polyoxyethylene stearate,
   (c) a condensation product of ethylene oxide with a long chain aliphatic alcohol, for example, heptadecaethyleneoxycetanol,
   (d) a condensation product of ethylene oxide with a partial ester derived from a fatty acid and a hexitol such as polyoxyethylene sorbitol monooleate, or
   (e) a condensation product of ethylene oxide with a partial ester derived from a fatty acid and a hexitol anhydride, for example polyoxyethylene sorbitan monooleate.

The aqueous suspensions may also contain one or more preservatives, for example, ethyl or n-propyl p-hydroxybenzoate; one or more coloring agents; one or more flavoring agents; and one or more sweetening agents such as sucrose, aspartame or saccharin.

Oily suspension may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents and flavoring agents may be added to provide a palatable oral preparation. These compositions may be preserved by the addition of an antioxidant such as ascorbic acid.

Dispersible powders and granules are suitable for the preparation of an aqueous suspension. They provide

the active ingredient in admixture with a dispersing or wetting agent, a suspending agent and one or more preservatives. Suitable dispersing or wetting agents and suspending agents are exemplified by those already mentioned above. Additional excipients, for example, those sweetening, flavoring and coloring agents described above may also be present.

The pharmaceutical compositions of the invention may also be in the form of oil-in-water emulsions. The oily phase may be a vegetable oil such as olive oil or arachis oils, or a mineral oil such as liquid paraffin or a mixture thereof. Suitable emulsifying agents may be (1) naturally-occurring gums such as gum acacia and gum tragacanth, (2) naturally-occurring phosphatides such as soy bean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Syrups and elixirs may be formulated with sweetening agents, for example, glycerol, propylene glycol, sorbitol, aspartame or sucrose. Such formulations may also contain a demulcent, a preservative and flavoring and coloring agents.

The pharmaceutical compositions may be in the form of a sterile injectable aqueous or oleagenous suspension. This suspension may be formulated according to known methods using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The disclosed cyclosporines may also be administered in the form of suppositories for rectal administration of the drug. These compositions can be prepared by mixing the drug with a suitable nonirritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the disclosed cyclosporines are employed.

Dosage levels of the order from about 0.05 mg to about 50 mg per kilogram of body weight per day are useful in the treatment of the above-indicated conditions (from about 2.5 mg to about 2.5 gms. per patient per day).

The amount of active ingredient that may be combined with the carrier materials to produce a single dosage form will vary depending upon the host treated and the particular, mode of administration. For example, a formulation intended for the oral administration of humans may contain from 2.5 mg to 2.5 gm of active agent compounded with an appropriate and convenient amount of carrier material which may vary from about 5 to about 95 percent of the total composition. Dosage unit forms will generally contain between from about 5 mg to about 500 mg of active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

In the case of conflict, the text of the application is controlling. Modifications and changes of the disclosed compounds and methods will be apparent to one skilled in the art. Such modifications and changes are intended to be encompassed by this disclosure and the claims appended hereto.

A further specific embodiment of the invention is represented by formula I:

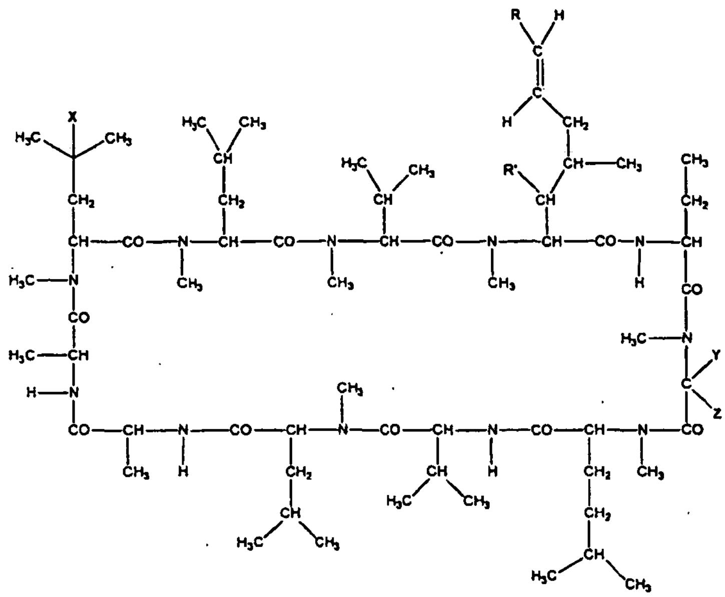

(I)

wherein R is

(i) a deuterium or
(ii) a saturated or unsaturated straight or branched aliphatic carbon chain of from 1 to 16 carbon atoms and optionally containing one or more deuterium atoms or an ester, ketone or alcohol of said carbon chain and optionally containing one or more substituents selected from halogen, nitro, amino, amido, aromatic, and heterocyclic, or
(iii) an aromatic or heterocyclic group containing one or more deuterium atoms, or
(iv) a methyl group and

X, Y, and Z are hydrogen or deuterium provided that if R is methyl then at least one of X, Y, or Z is deuterium and wherein R' is an OH or acetate or other ester or is an O and together with a carbon adjacent to a double bond on amino acid 1 forms a heterocyclic ring.

R may be, for example, a saturated or unsaturated aliphatic carbon chain of from 2 to 3 carbons containing one or more deuterium atoms.

A further specific embodiment of the invention is a pharmaceutical composition comprising a cyclosporine. A derivative of the invention or a pharmaceutically acceptable salt thereof and a pharmaceutically acceptable carrier. This pharmaceutical composition may be for use in immunosuppression.

## Claims

**1.** A cyclosporin A derivative represented by the formula:

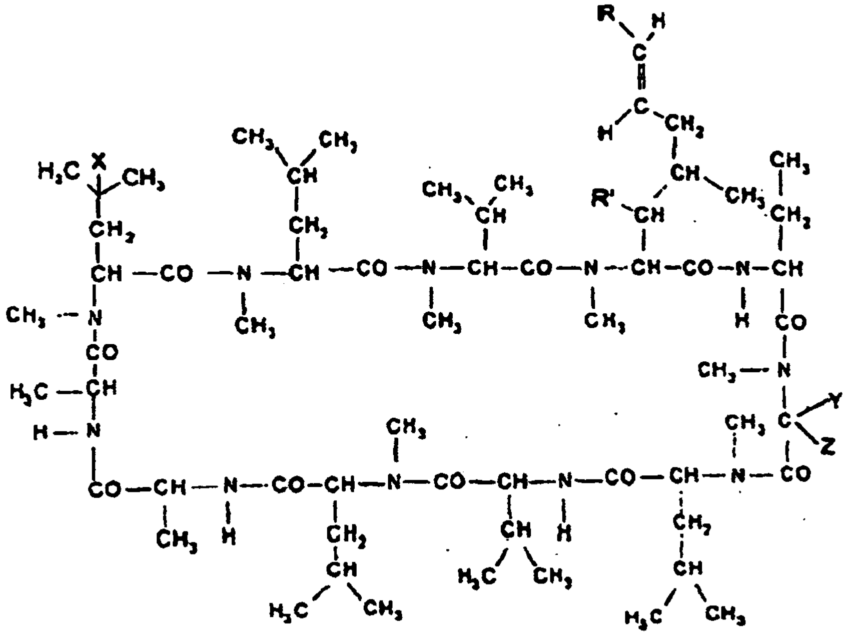

(I)

or a pharmaceutically acceptable salt thereof, wherein:

X=Y=Z=H;
R is a saturated or unsaturated aliphatic carbon chain of 2 or 3 carbon atoms containing one or more deuterium atoms in place of hydrogen; and
R'=OH.

2. The cyclosporin derivative of claim 1, or a pharmaceutically acceptable salt thereof, for use as a medicament.

3. The cyclosporin derivative of claim 1, or a pharmaceutically acceptable salt thereof, for use in providing immunosuppression in a subject.

4. The cyclosporin derivative of claim 1, or a pharmaceutically acceptable salt thereof, for use in preventing or ameliorating autoimmune disease in a subject.

5. Use of the cyclosporin derivative of claim 1, or a pharmaceutically acceptable salt thereof, in the manufacture of a medicament for providing immunosuppression in a subject.

6. Use of the cyclosporin derivative of claim 1, or a pharmaceutically acceptable salt thereof, in the manufacture of a medicament for preventing or ameliorating autoimmune disease in a subject.

7. A pharmaceutical composition comprising the cyclosporin derivative of claim 1, or 8 pharmaceutically acceptable salt thereof, and a pharmaceutically acceptable carrier.

8. A cyclosporin A derivative or a pharmaceutically acceptable salt thereof, wherein one or more hydrogen atoms in the amino acid position selected from the group consisting of 1 and 9 or combinations thereof are substituted with a deuterium atom, and wherein said cyclosporin A derivative is optionally chemically substituted at the amino acid 9 position.

9. The cyclosporin A derivative of claim 8, wherein the cyclosporin A derivative is represented by the formula:

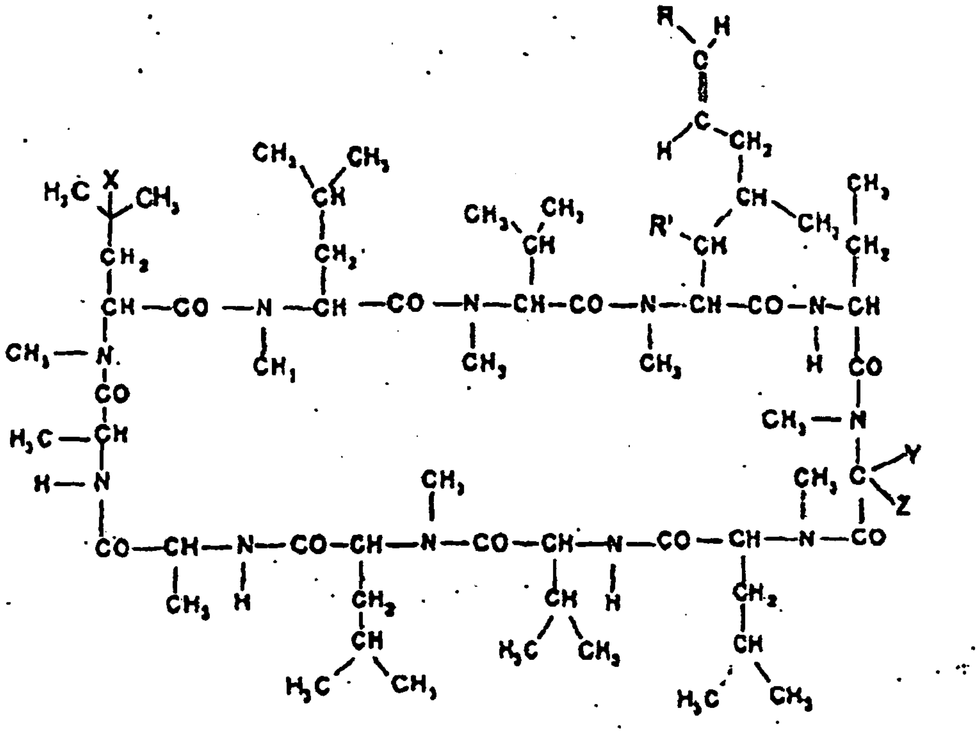

(I)

or a pharmaceutically acceptable salt thereof, wherein:

R is

(i) deuterium;
(ii) a saturated or unsaturated straight or branched aliphatic carbon chain of from 1 to 16 carbon atoms, or an ester, ketone or alcohol of said carbon chain, wherein said carbon chain is optionally substituted with one or more deuterium, halogen, nitro, amino, amido, aromatic, or heterocyclic groups, or
(iii) an aromatic or heterocyclic group containing one or more deuterium atoms;

X, Y and Z are hydrogen or deuterium, provided that if R is methyl then at least one of X, Y and Z is deuterium; and R' is an OH, acetate, or other ester, or R' is an O atom that, together with a carbon adjacent to a double bond on a side chain of the amino acid to which R' is bonded, forms a heterocyclic ring.

**10.** A cyclosporin A derivative represented by the formula:

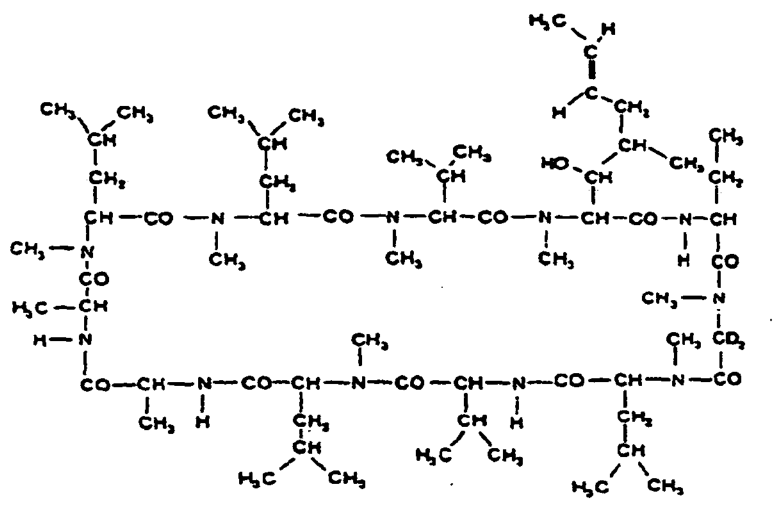

or a pharmaceutically acceptable salt thereof.

**11.** The cyclosporin derivative of claim 8, 9 or 10, or a pharmaceutically acceptable salt thereof, for use as a medicament.

**12.** The cyclosporin derivative of claim 8, 9 or 10, or a pharmaceutically acceptable salt thereof, for use in providing immunosuppression in a subject.

**13.** The cyclosporin derivative of claim 8, 9 or 10, or a pharmaceutically acceptable salt thereof, for use in preventing or ameliorating autoimmune disease in a subject.

**14.** Use of the cyclosporin derivative of claim 8, 9 or 10, or a pharmaceutically acceptable salt thereof, in the manufacture of a medicament for providing immunosuppression in a subject.

**15.** Use of the cyclosporin derivative of claim 8, 9 or 10, or a pharmaceutically acceptable salt thereof, in the manufacture of a medicament for preventing or ameliorating autoimmune disease in a subject.

**16.** A pharmaceutical composition comprising the cyclosporin derivative of claim 8, 9 or 10, or a pharmaceutically acceptable salt thereof, and a pharmaceutically acceptable carrier.

**Patentansprüche**

**1.** Cyclosporin A-Derivat der Formel:

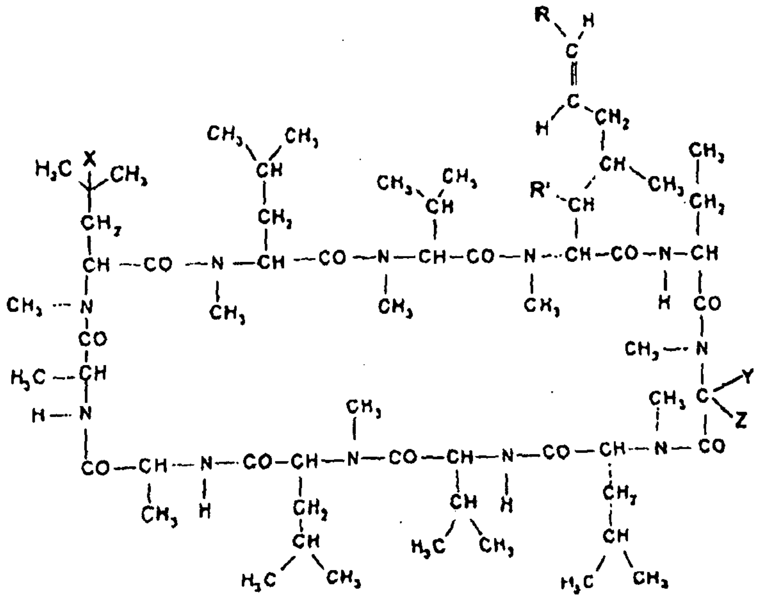

(I)

oder dessen pharmazeutisch annehmbares Salz, worin ist

X gleich Y gleich Z gleich H;
R eine gesättigte oder ungesättigte aliphatische Kohlenstoffkette mit 2 oder 3 Kohlenstoffatomen, die ein oder mehrere Deuteriumatome anstelle von Wasserstoff enthält; und
R' gleich OH.

**2.** Cyclosporin A-Derivat nach Anspruch 1 oder dessen pharmazeutisch annehmbares Salz in der Verwendung als Arzneimittel.

**3.** Cyclosporin-Derivat nach Anspruch 1 oder dessen pharmazeutisch annehmbares Salz in der Verwendung: Herstellen einer Immunsuppression in einer Person.

**4.** Cyclosporin-Derivat nach Anspruch 1 oder dessen pharmazeutisch annehmbares Salz in der Verwendung: Verhindern oder Lindern einer Autoimmunerkrankung in einer Person.

**5.** Verwendung des Cyclosporin-Derivats von Anspruch 1 oder dessen pharmazeutisch annehmbares Salz in der Herstellung eines Arzneimittels zum Erzeugen einer Immunsuppression in einer Person.

**6.** Verwendung des Cyclosporin-Derivats von Anspruch 1 oder dessen pharmazeutisch annehmbaren Salz in der Herstellung eines Arzneimittels zum Verhindern oder Lindern einer Autoimmunerkrankung in einer Person.

**7.** Pharmazeutische Zusammensetzung, enthaltend das Cyclosporin-Derivat von Anspruch 1 oder dessen pharmazeutisch annehmbares Salz sowie einen pharmazeutisch annehmbaren Träger.

**8.** Cyclosporin A-Derivat oder dessen pharmazeutisch annehmbares Salz, worin ein oder mehrere Wasserstoffatome in einer Aminosäureposition, ausgewählt aus 1 und 9 oder Kombinationen davon, durch Deuteriumatome ersetzt sind und wobei das Cyclosporin A-Derivat wahlweise auf der Aminosäureposition 9 chemisch substituiert ist.

**9.** Cyclosporin A-Derivat nach Anspruch 8 der Formel

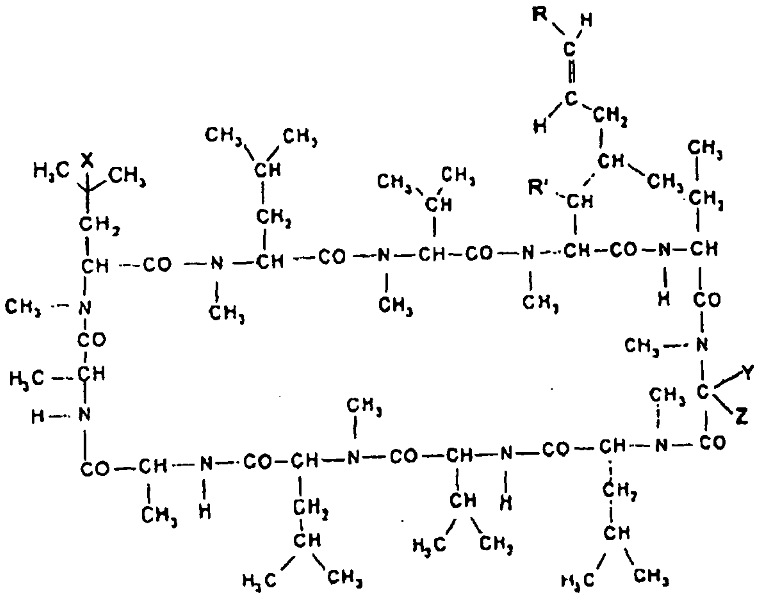

(I)

oder ein pharmazeutisch annehmbares Salz davon, worin ist:

R gleich

(i) Deuterium,
(ii) eine gesättigte oder ungesättigte lineare oder verzweigte aliphatische Kohlenstoffkette mit 1 bis 16 Kohlenstoffatomen, oder ein Ester, ein Keton oder ein Alkohol einer solchen Kohlenstoffkette, wobei die Kohlenstoffkette wahlweise mit ein oder mehreren Deuterium-, Halogen-, Nitro-, Amido-, Amino-, Aromat- oder Heterozyklo-Gruppen substituiert ist, oder
(iii) eine aromatische oder heterozyklische Gruppe mit einem oder mehreren Deuteriumatomen ist;

X, Y und 7 gleich Wasserstoff oder Deuterium, vorausgesetzt, wenn R gleich Methyl, dass mindestens eine Stelle aus X, Y und Z Deuterium ist; und
R' gleich OH, Acetat oder ein anderer Ester, oder gleich ein Sauerstoffatom, das zusammen mit einem Kohlenstoff nächst einer Doppelbindung auf einer Seitenkette der Aminosäure, an die R' gebunden ist, einen heterozyklischen Ring bildet.

**10.** Cyclosporin A-Derivat der Formel:

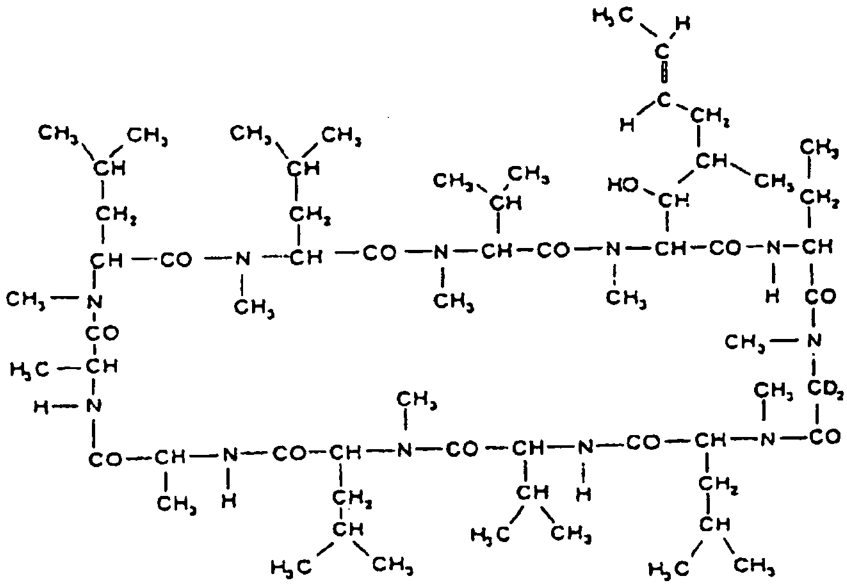

oder dessen pharmazeutisch annehmbares Salz.

11. Cyclosporin-Derivat nach Anspruch 8, 9 oder 10 oder ein pharmazeutisch annehmbares Salz davon zur Verwendung als Arzneimittel.

12. Cyclosporin-Derivat nach Anspruch 8, 9 oder 10 oder dessen pharmazeutisch annehmbares Salz in der Verwendung: Herstellen einer Immunsuppression in einer Person.

13. Cyclosporin-Derivat nach Anspruch 8, 9 oder 10 oder dessen pharmazeutisch annehmbares Salz in der Verwendung: Verhindern oder Lindern einer Autoimmunerkrankung in einer Person.

14. Verwendung des Cyclosporin-Derivats von Anspruch 8, 9 oder 10 oder dessen pharmazeutisch annehmbaren Salzes in der Herstellung eines Arzneimittels zum Erzeugen einer Immunsuppression in einer Person.

15. Verwendung des Cyclosporin-Derivats von Anspruch 8, 9 oder 10 oder dessen pharmazeutisch annehmbaren Salzes in der Herstellung eines Arzneimittels zum Verhindern oder Lindern einer Autoimmunerkrankung in einer Person.

16. Pharmazeutische Zusammensetzung, enthaltend das Cyclosporin-Derivat von Anspruch 8, 9 oder 10 oder dessen pharmazeutisch annehmbares Salz sowie einen pharmazeutisch annehmbaren Träger.

## Revendications

1. Dérivé de cyclosporine A représenté par la formule:

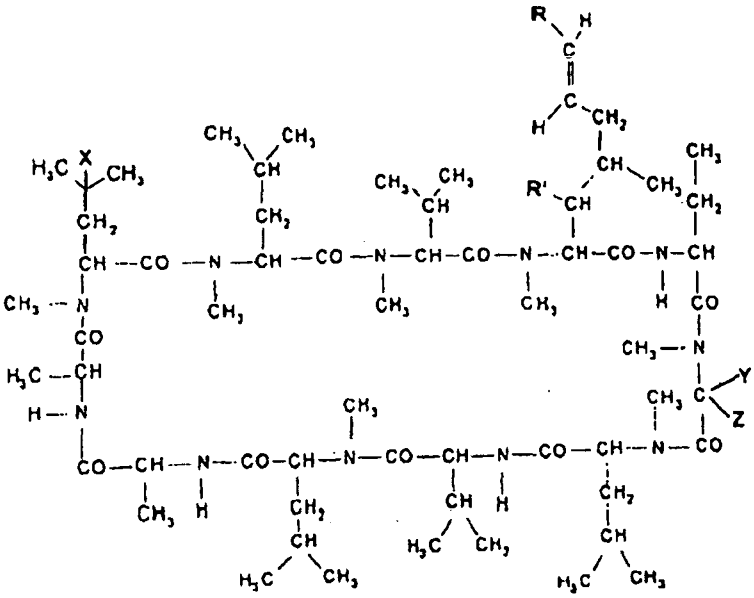

(I)

ou l'un de ses sels acceptables pharmaceutiquement, dans lequel:

X=Y=Z=H;
R est une chaîne carbonée aliphatique, saturée ou insaturée de 2 ou 3 atomes de carbone comprenant un ou plusieurs atomes de deutérium au lieu d'hydrogène ; et
R'=OH.

2. Dérivé de cyclosporine selon la revendication 1, ou l'un de ses sels acceptables pharmaceutiquement, à utiliser comme médicament.

3. Dérivé de cyclosporine selon la revendication 1, ou l'un de ses sels acceptables pharmaceutiquement, à utiliser pour donner de l'immunosuppression à un sujet.

4. Dérivé de cyclosporine selon la revendication 1, ou l'un de ses sels acceptables pharmaceutiquement, à utiliser pour prévenir ou soulager une maladie auto-immune chez un sujet.

5. Utilisation du dérivé de cyclosporine de la revendication 1, ou de l'un de ses sels acceptables pharmaceutiquement, dans la fabrication d'un médicament pour donner de l'immunosuppression chez un sujet.

6. Utilisation du dérivé de cyclosporine de la revendication 1, ou de l'un de ses sels acceptables pharmaceutiquement, dans la fabrication d'un médicament pour prévenir ou soulager une maladie auto-immune chez un sujet.

7. Composition pharmaceutique comprenant un dérivé de cyclosporine selon la revendication 1, ou l'un de ses sels acceptables pharmaceutiquement, et un véhicule acceptable pharmaceutiquement.

8. Dérivé de cyclosporine A ou l'un de ses sels aceptables pharmaceutiquement, dans lequel un ou plusieurs atomes d'hydrogène dans les positions d'acides aminés, sélectionnées du groupe comprenant 1 et 9 ou de combinaisons de ces positions, sont substitués par un atome de deutérium et dans lequel le dérivé de cyclosporine A est facultativement substitué chimiquement dans la position d'acide aminé 9.

9. Dérivé de cyclosporine A selon la revendication 8, représenté par la formule:

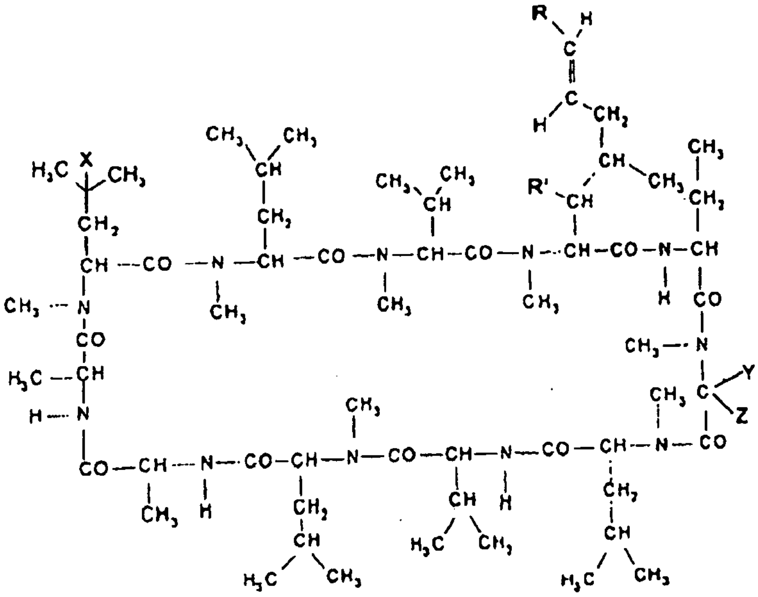

(I)

ou l'un de ses sels acceptables pharmaceutiquement, dans lequel:

R est

(i) deutérium;

(ii) une chaîne carbonnée aliphatique, saturée ou insaturée, linéaire ou branchée, avec 1 à 16 atomes de carbone, ou bien un ester, une cétone ou un alcohol d'une telle chaîne carbonnée, où la chaîne carbonnée est substituée au choix avec un ou plusieurs atomes deutérium ou halogène ou avec un ou plusieurs groupes nitro, amido, amino, aromatiques ou hétérocycliques; ou

(iii) un ou plusieurs groupes aromatiques ou hétérocycliques avec un ou plusieurs atomes de deutérium;

X, Y et Z sont hydrogène ou deutérium, à condition que, si R est méthyle, au moins l'un d'entre X, Y et Z est deutérium ; et

R' est OH, acétate ou un autre ester, ou R' est un atome d'oxygène qui forme, avec un atome de carbone situé à côté d'une double liaison sur une branche de l'acide aminé lié à R', un hétérocycle.

**10.** Dérivé de cyclosporine A représenté par la formule:

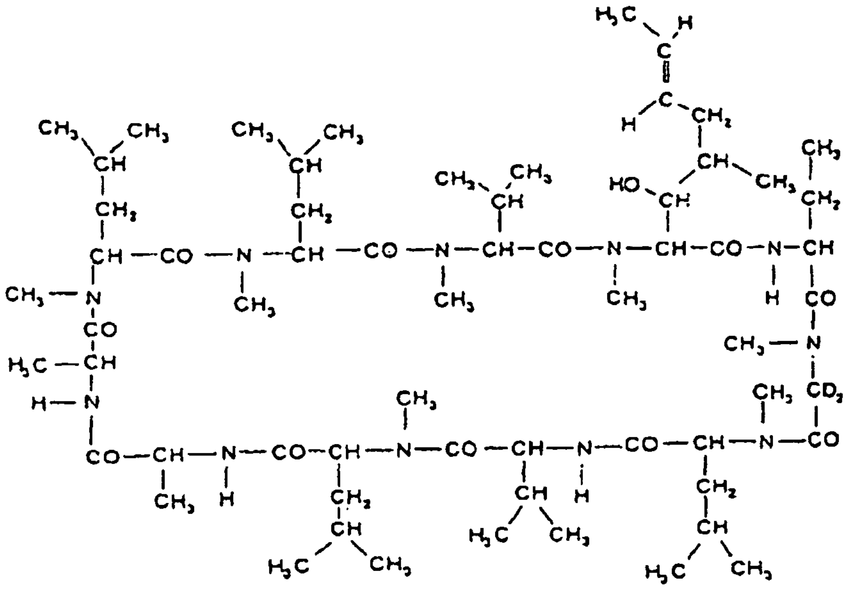

ou l'un de ses sels acceptables pharmaceutiquement.

11. Dérivé de cyclosporine selon la revendication 8, 9 ou 10, ou l'un de ses sels acceptables pharmaceutiquement, à utiliser comme médicament.

12. Dérivé de cyclosporine selon la revendication 8, 9 ou 10, ou l'un de ses sels acceptables pharmaceutiquement, à utiliser pour donner de l'immunosuppression à un sujet.

13. Dérivé de cyclosporine selon la revendication 8, 9 ou 10, ou l'un de ses sels acceptables pharmaceutiquement, à utiliser pour prévenir ou soulager une maladie auto-immune chez un sujet.

14. Utilisation du dérivé de cyclosporine de la revendication 8, 9 ou 10, ou de l'un de ses sels acceptables pharmaceutiquement, dans la fabrication d'un médicament pour donner de l'immunosuppression chez un sujet.

15. Utilisation du dérivé de cyclosporine de la revendication 8, 9 ou 10, ou de l'un de ses sels acceptables pharmaceutiquement, dans la fabrication d'un médicament pour prévenir ou soulager une maladie auto-immune chez un sujet.

16. Composition pharmaceutique comprenant un dérivé de cyclosporine selon la revendication 8, 9 ou 10, ou l'un de ses sels acceptables pharmaceutiquement, et un véhicule acceptable pharmaceutiquement.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 06136097 P
- US 4117118 A, E. Harri
- GB 2206199 A
- US 4108985 A
- US 4210581 A
- US 4220641 A
- US 4288431 A
- US 4289851 A
- US 4384996 A
- US 4396542 A
- US 4639434 A
- US 4681754 A
- US 4703033 A
- US 4798823 A
- WO 9526325 A
- US 4256108 A
- US 4160452 A
- US 4265874 A


### Non-patent literature cited in the description


- **LAWEN et al.** *J. Antibiotics,* 1989, vol. 42, 1283
- **TRABER et al.** *Helv. Chim. Acta,* 1987, vol. 70, 13
- **VON WARTBURG ; TRABER.** *Prog. Med. Chem,* 1988, vol. 25, 1
- **A. RUEGGER et al.** *Helv. Chim. Acta,* 1976, vol. 59, 1075
- **M. DREYFUSS et al.** *J. Appl. Microbiol.,* 1976, vol. 3, 125
- **T. J. PETCHER et al.** *Helv. Chim. Acta,* 1976, vol. 59, 1480
- **R. TRABER et al.** *ibid,* 1977, vol. 60, 1247
- **R. TRABER et al.** *Helv. Chim. Acta,* 1977, vol. 60, 1568
- **E, F, G, H, I: EIDEM.** *ibid,* 1982, vol. 65, 1655
- **J. F. BOREL et al.** *Agents Actions,* 1976, vol. 6, 468
- **EIDEM.** *Immunology,* 1977, vol. 32, 1017
- **R. Y. CALNE.** *Clin. Exp. Immunol,* 1979, vol. 35, 1
- **R. Y. CALNE et al.** *Lancet,* 1978, vol. 2, 1323
- **R. L. POWLES et al.** *ibid,* 1327
- **R. L. POWLES et al.** *ibid,* 1980, vol. 1, 327
- **D. J. WHITE et al.** *Transplantation,* 1979, vol. 27, 55
- **M. Y. GORDON ; J. W. SINGER.** *Nature,* 1979, vol. 279, 433
- **P. J. TUTSCHKA et al.** *Blood,* 1983, vol. 61, 318
- **PFLUGL, G. ; KALLEN, J. ; SCHIRMER, T ; JANSONIUS, J.N ; ZURINI, M.G.M. ; WALKINSHAW, M.D.** *Nature,* 1993, vol. 361, 91-94
- **LIU, J. ; FARMER, J.D. ; LANE, W.S. ; FRIEDMAN, J. ; WEISSMAN, I. ; SCHREIBER, S.L.** *Cell,* 1991, vol. 66, 807-15
- **SWANSON, S.K. ; BORN, T ; ZYDOWSKY, C.D. ; CHO, H. ; CHANG, H.Y. ; WALSH, C.T.** *Proc. Natl. Acad. Sci.USA,* 1992, vol. 89, 3686-90
- **LIU, J. ; FARMER, J.D ; LANE, W.S ; FRIEDMAN, J. ; WEISSMAN, I. ; SCHREIBER, S.L.** *Cell,* 1991, vol. 66, 807-15
- **ETZKORN, F. A. ; CHANG, Z ; STOLZ, L.A. ; WALSH, C.T.** *Biochemistry,* 1994, vol. 33, 2380-2388
- **LIU, J. ; FARMER, J.D ; LANE, W.S. ; FRIEDMAN, J. ; WEISSMAN, I. ; SCHREIBER, S.L.** *Cell,* 1991, vol. 66, 807-15
- **WENGER et al.** *Cyclosporine: Chemistry, Structure-Activity Relationships and Mode of Action, Progress in Clinical Biochemistry and Medicine,* 1986, vol. 2, 176
- **R. TRABER et al.** *J. Antibiotics,* 1989, vol. 42, 591
- **H. KOBEL ; R. TRABER.** *Directed Biosynthesis of Cyclosporins, European J. Appln. Microbiol Biotechnol.,* 1982, vol. 14, 237B240
- **D. SEEBACH.** *Helvetica Chimica Acta,* 1993, vol. 76, 1564-1590
- **KOBEL H et al.** Experientia. Birkhauser Verlag, 1983, vol. 39, 873-876
- **PETTERSEN et al.** *Anticancer Res,* 1992, vol. 12, 33
- **BLAKE et al.** *J. Pharm. Sci.,* 1975, vol. 64 (3), 367-391
- **FOSTER et al.** Advances in Drug Research. Academic press, vol. 14, 2-36
- **FRUMAN et al.** *A Proc Natl Acad Sci USA,* 1992